# EUROPEAN PATENT SPECIFICATION

(11) **EP 2 332 530 B1**
(45) Date of publication and mention of the grant of the patent: **08.10.2014**
(21) Application number: 08773142.8
(22) Date of filing: 08.07.2008
(51) Int. Cl.: A61K 31/192, A61K 9/08, A61K 9/107, A61K 9/20, A61K 9/48, A61K 9/14, A61P 25/28, A61P 43/00

(54) **THE USE OF POTASSIUM 2-(HYDROXYPENTYL) BENZOATE IN THE MANUFACTURE OF MEDICAMENTS FOR PREVENTING AND/OR TREATING SENILE DEMENTIA**
VERWENDUNG VON KALIUM-2-(HYDROXYPENTYL) BENZOAT BEI DER HERSTELLUNG VON ARZNEIMITTELN ZUR PRÄVENTION UND/ODER BEHANDLUNG VON ALTERSDEMENZ
UTILISATION DE 2-(HYDROXYPENTYL) BENZOATE DE POTASSIUM DANS LA FABRICATION DE MÉDICAMENTS DESTINÉS À LA PRÉVENTION/AU TRAITEMENT DE LA DÉMENCE SÉNILE

(43) Date of publication of application: 15.06.2011
(73) Proprietor: INSTITUTE OF MATERIA MEDICA, CHINESE ACADEMY OF MEDICAL SCIENCES, Beijing 100050 (CN)
(72) Inventor: WANG, Xiaoliang, Beijing 100050 (CN); ZHAO, Wanhong, Beijing 100050 (CN); YANG, Jinghua, Beijing 100050 (CN); WANG, Ling, Beijing 100050 (CN); LI, Jiang, Beijing 100050 (CN); XU, Shaofeng, Beijing 100050 (CN); FENG, Nan, Beijing 100050 (CN); MA, Shiping, Beijing 100050 (CN)
(74) Representative: Held, Stephan
(86) International application number: PCT/CN2008/071588
(87) International publication number: WO 2010/003287

(56) References cited:
- EP-A1- 1 508 561
- CN-A- 1 382 682
- CN-A- 1 560 018
- CN-A- 1 594 270
- CN-A- 101 054 346
- CN-A- 101 054 346

## Description

### Field of the invention:

The invention concerns prevention or treatment of neurological diseases, in particular of aged dementia. It relates to the use of dl-PHPB for the prevention or treatment of aged dementia, or a symptom thereof, wherein said use is for the prevention, amelioration or treatment of memory loss, cognitive dysfunction, slow thinking and spatial disorientation. The aged dementia includes Alzheimer's Disease, vascular dementia and a combination of both. It also relates to pharmaceutical compositions for said prevention or treatment of aged dementia, which contain dl-PHPB and pharmaceutically acceptable excipients or carriers. Pharmaceutical compositions of dl-PHPB for the manufacture are chosen from solution, suspension, emulsion, pill, capsule, powder, control or continuous release preparation. The invention also relates to the claimed compounds for use in methods for the prevention or treatment of said aspects of aged dementia, including providing an effective dosage of dl-PHPB or pharmaceutical compositions containing dl-PHPB to patients. The approach of providing dl-PHPB to patients can include external oral, local, intracutaneously, intramuscular, peritoneal, subcutaneous, intranasal, and so on.

### Background of the invention:

Aged dementia includes Alzheimer's Disease (AD), vascular dementia (VD), and a combination of both. Aged dementia is a neurodegenerative disorder that is characterized by a progressive cognitive impairment and memory damage. This disease accounts for most dementias in old people, in particular aged above 60. In this disease the ability to remember, think, understand, communicate, and control behavior progressively declines because brain tissue degenerates. Many neurons in these brain regions contain large neurofibrillary tangles together with amyloid beta depositions. In China, the prevalence rate of aged dementia accounts for 4% in those above 65 years old. In the world, there are about fifty million patients having aged dementia. VD is a neurodegenerative disorder that is characterized by a cerebrovascular disease. In Europe and America, patients of VD account for 10-20% of aged dementia. In Asia, the incidents of VD are higher, such as Japan and China. China is entering the era of aging, with patients of aged dementia increasing year by year. Long term cerebral-ischemia is the main cause to form VD. Patients of aged dementia suffer themselves, and also impose a great burden for their family and society. There is no effective drug for treatment of aged dementia at present. Therefore, it is important to identify and develop effective drugs to control or treat progressive of AD and VD.

Potassium 2-(1-Hydroxypentyl)-benzoate (dl-PHPB), derived from 3-n-butylphthalide (NBP), is a newly synthesized compound that is under development as a therapeutic drug for cerebral ischemia. dl-PHPB was provided by the Department of Synthetic Pharmaceutical Chemistry of Chinese Academy of Medical Sciences with a purity of 99.9%. dl-PHPB is characterized by X-crystal diffraction, NMR, MS, infrared spectra, and HPLC-UV The chemical structure of this compound is shown in Fig.1. The preparation of dl-PHPB was described in PCT/CN02/1382682, entitled "novel salts of 2-(α-hydroxypentyl) benzoic acid, the methods for preparation and the application of these salts".

CN101054346 describes the enantiomerically pure S derivative of 2-(α-hydroxypentyl)benzoic acid as a medication for the treatment of cerebral ischemia. In the investigations underlying this document, the effectiveness of (S)-potassium 2-(α-hydroxypentyl)benzoate was evaluated by the rat MCAO (middle cerebral arterial occlusion) model by administering the component to an MCAO rat, sacrificing the rat and the evaluating cerebral tissue affected by the occlusion. In these investigations it was found that the administration of a single dose (100mg/kg of (S)-potassium 2-(α-hydroxypentyl)benzoate prior to the surgery inflicting the arterial occlusion resulted in a significant reduction of the infarct size compared to a control group which was not treated with (S)-potassium 2-(α-hydroxypentyl)benzoate.

There has been no report of using dl-PHPB for prevention or treatment of aged dementia thus far.

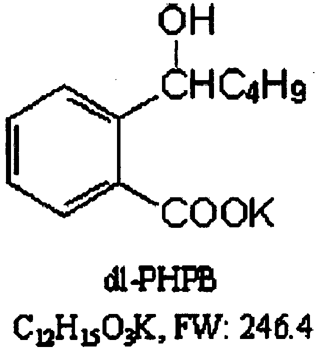

### Content of the invention:

In a first and in a second aspect, the invention relates to the subject-matter of present claims 1 and 10.

As used herein, aged dementia includes Alzheimer's Disease (AD), vascular dementia (VD), and conditions having a combination of both AD and VD. The symptoms of aged dementia can include memory impairment, impaired cognition, impaired thought process and impaired spatial orientation.

In the third aspect, the invention relates to dl-PHPB for prevention of aged dementia as claimed wherein earliest changes in aged dementia occur in he ability to remember, think, understand, communicate, and wherein control behavior progressively declines.

In the fourth aspect, the invention relates to dl-PHPB for use according to the first aspect for improving oxidative stress damage, function of cholinergic neurons, protecting neuron and enhancing brain-derived neurotrophic factor (BDNF) in brain. The invention related to dl-PHPB for improving oxidative stress damage, i.e. to decrease lipid peroxides (Malondialdehyde, MDA)), and restore the balance of oxidation. The invention related to dl-PHPB for improving neurons function, i.e. to enhance the activity of choline acetyltransferase (ChAT) and decrease the activity of acetylcholinesterase.

In the invention, the preferred mammalian chosen is human.

The aged dementia is a neurodegenerative disorder that is characterized by a cognition defection, in particular short-term memory, and space sentience progressively declining. Aged dementia is induced by different cause, such as VD, neurofibrillary tangles together with amyloid beta depositions, and other age-related causes. The invention adopted three dementia models: bilateral cervical carotid artery occlusion to simulate clinical VD, cognitive impairment induced by intracerebroventricular infusion of amyloid-beta (25-35) peptide in rats used to simulate clinical AD, and accelerated aging mice model (SAMP8) to simulate aging. dl-PHPB improved the learning and memory capability by the Morris water maze test, the Y type water maze test and step down test in rats or mice. The results showed dl-PHPB significantly improved cognitive impairment in bilateral cervical carotid artery occlusion (2-VO) model, intracerebroventricular infusion of amyloid-beta (25-35) peptide model, and accelerated aging mice model (SAMP8).

In summary, dl-PHPB significant improved cognitive impairment in the three dementia models. The results showed dl-PHPB significantly improved abnormity of brain tissue induced by long-term cerebral-ischemia, decreased activation of astrocytes, and furthermore protected neurons; and exerted reduction of level of MDA, inhibition of oxidative stress damage; enhanced the activity of ChAT, increasing level of acetylcholine and improving the learning and memory capability; increased the level of BNDF after long-term cerebral-ischemia. Therefore, dl-PHPB might be a potential drug for the prevention or treatment of aged dementia.

### Detailed description of the invention.

The scope of the invention is limited by the scope of the appended claims. The present invention is based on the finding that in a long-term cerebral-ischemia model, in which the short-term memory, space sentience capability had been improved by dl-PHPB. VD is a neurodegenerative disease induced by cerebrovascular disease, following cerebral artery occlusion, or low infusion, and lacunal cerebral stroke. The reduction of blood stream in brain related to aged dementia. Long-term cerebral-ischemia is contributed to decrease utilization of oxygen, glucose and other necessary metabolites, furthermore inducing oxidative stress damage, reduction of mitochondrion function, reduction of neuron biosynthesis, inhibition of transmission of synapse, and forming neurodegenerative changes. VD is a neurodegenerative disease , that is characterized by a cognition defection, in particular short-term memory, space sentience progressively decline.

The Morris water maze test is a typical experiment for detecting short-term memory, space sentience learning capability of animals. In the invention, the effect of dl-PHPB for improving short-term memory, space sentience learning capability was detected by bilateral cervical carotid artery occlusion (2-VO) model in rats. The results indicate that dl-PHPB significantly improves short-term memory, space sentience learning capability of 2-VO model in rats. At one month after the onset of bilateral cervical carotid artery occlusion, the rats were tested by the escape latencies of place navigation testing and the times of crossing the exact position of the former platform of probe trial testing in Morris water maze test in which spatial learning and memory capability were assessed.

In place navigation testing, the escape latencies, search strategy, and speed of swimming were investigated in Morris water maze test. The results indicate that dl-PHPB significantly improves learning and memory capability in rats by onset of bilateral cervical carotid artery occlusion. The effect of decreasing the escape latencies in 2-VO rat model, showed that dl-PHPB significantly improves short-term memory, space sentience learning capability of 2-VO rat model. The results of search strategy showed that rats treated with dl-PHPB significantly increased the times of straightaway and tendency strategy, in which indicating dl-PHPB significantly increase space sentience learning capability of 2-VO rat model. The speed of rats treated with dl-PHPB was not significantly different, compared to the rats treated with saline, in which indicating the method for detecting effect of dl-PHPB improving memory and learning capability was not influenced by the physical strength of rats. The experiments results indicated that dl-PHPB could prevent and treat aged dementia, in particular improved spatial learning and short-term memory capability of vascular dementia disease.

After place navigation testing, the platform of probe trial testing were investigated in Morris water maze test, in which the retain time of target quadrant, and time of first crossing the platform location was recorded when the platform was removed to detect the memory capability of rats for the platform. The results showed the rats treated with dl-PHPB significantly increased the retain time of target quadrant, and decreased the time of first crossing the platform location, compared to the mice treated with saline. The results indicate that dl-PHPB improves the spatial learning capability of 2-VO rat model. The experiments results indicated dl-PHPB could prevent and treat aged dementia, in particular improved spatial learning and memory capability of vascular dementia disease.

For elucidating the mechanism of dl-PHPB for prevention and treatment of vascular dementia disease, the index related to oxidant stresses damage and cholinergic system were detected, in particular the activity of superoxide dismutasen (SOD), level of MDA and activity of ChAT by measured in brain tissue in rats treated with dl-PHPB.

SOD is a major antioxidants enzyme in vivo, with radical scavenging action and decreasing oxidant damage. MDA is a major production of super oxidant. The activity of SOD in brain reflected the capacity of anti-oxygenation, whereas level of MDA reflected state of peroxidation. At one month after the onset of bilateral cervical carotid artery occlusion, the activity of SOD and level of MDA significantly increased in cortex, compared to the rats treated with saline. The results indicated that dl-PHPB could improve disorder of the capacity of anti-oxygenation in brain, decrease production of lipid peroxidation, restore the balance of oxidation. dl-PHPB might be used in the prevention, amelioration and treatment of aged dementia, i.e. as an antioxidant and free radical scavenger in brain tissue, wherein that said use is for the prevention, amelioration and treatment of memory loss, cognitive dysfunction, slow thinking or spatial disorientation.

Acetylcholine is a major neurotransmitter, inducing signal transduction of cholinergic neurons and related to learning and memory. ChAT is a acetylcholine synthesis enzyme, the activity of ChAT reflected the function of cholinergic neurons. At one month after the onset of bilateral cervical carotid artery occlusion, the activity of ChAT significantly decreased in hippocampus. The rats treated with dl-PHPB for 21 days, significantly increased the activity of ChAT, and improved the function of cholinergic neurons. The results indicated that dl-PHPB might be used in the prevention, amelioration and treatment of aged dementia to enhance function of cholinergic neurons.

The behavior in rats has been changed by bilateral cervical carotid artery occlusion, following activation of glia cell and change of white matter and grey matter. Moreover, for further elucidation of the mechanism of dl-PHPB for prevention and treatment of aged dementia disease, pathology of rats treated with dl-PHPB was investigated by pathology and immunohistochemistry. The indexes includes HE and Klüver-Barrera stained, Glial fibrillary acidic protein (GFAP), and BDNF. The results showed that the disorder of neuron in CA1and CA3 sub region was significantly improved in rats treated by dl-PHPB. The result of experiments indicated dl-PHPB could protect and treat the damage of cortex and hippocampus by bilateral cervical carotid artery occlusion.

K-B stain could reflect to the complete of myelination in neurons and pathological changes of nerve fiber. The vacuole formation of corpus callosum and disturbance of optic tracts in rats treated by dl-PHPB was significantly improved, compared to the rats treated with saline. The result of experiments indicated that dl-PHPB could protect the damage of corpus callosum and optic tracts by bilateral cervical carotid artery occlusion.

The effect of GFAP in glia cell was detected in rat treated with dl-PHPB by bilateral cervical carotid artery occlusion. Disturbances of brain tissue may be the basic of spatial learning and memory capability insult by bilateral cervical carotid artery occlusion. The earliest phase of damage in brain tissue is white matter, following increase of astrocytes and activation of microglia. Glial fibrillary acidic protein (GFAP) is an intermediate filament (IF) protein that is thought to be specific for astrocytes. In the invention, cortex, corpus callosum, optic tracts, and hippocampus subregion was chosen to evaluate the effect of dl-PHPB. In cortex, the express of GFAP in rats treated with saline increased, compared with control group. The express of GFAP in rats treated by dl-PHPB for 21 days was significantly decreased, compared to the rats treated with saline. In hippocampus, the express of GFAP in rats treated with saline significantly increased, compared with control group. The express of GFAP in rats treated by dl-PHPB for 21 days was also significantly decreased, compared to the rats treated with saline. In corpus callosum, the express of GFAP in rats treated with saline was no significant different, compared to control rats. The express of GFAP in rats treated by dl-PHPB for 21 days was decreased, compared to the rats treated with saline. In optic tracts, the express of GFAP in rats treated with saline increased, compared with control group. The express of GFAP in rats treated by dl-PHPB for 21 days was significantly decreased, compared to the rats treated with saline. In the above-mentioned experiment, dl-PHPB could significant improve the damage of brain tissue by bilateral cervical carotid artery occlusion, decreased the activity of astrocytes, particular in hippocampus, optic tracts, and cortex. The results of HE and K-B stain, expresses of GFAP indicated dl-PHPB could be used for preparation the drug to protect neuron.

In the invention, the BDNF was detected in brain tissue in rat treated with dl-PHPB by bilateral cervical carotid artery occlusion.BDNF acts on certain neurons of the central nervous system and the peripheral nervous system, helping to support the survival of existing neurons and encourage the growth and differentiation of new neurons and synapses. In the brain, it is active in the hippocampus, cortex, and basal forebrain-areas vital to learning, memory, and higher thinking. BDNF itself is important for long-term memory. The expression of BDNF was increased in the earliest of cerebral-ischemia, and decreased at post-ischemia for 24h. According to the results of immunohistochemistry, the expression of BDNF in cortex or hippocampus of vehicle group was significantly decreased, compared to control group. The expression of BDNF in cortex or hippocampus of rats treated by dl-PHPB for 21 days was significantly increased, compared to the rats treated with saline. The results showed dl-PHPB could increase the expresses of BDNF in brain tissue of rats by bilateral cervical carotid artery occlusion. The results of expression of BDNF indicated dl-PHPB could be used for the preparation of a drug to protect neurons.

In the invention, aged dementia rat model was induced by beta amyloid. dl-PHPB could improve the memory and space-learning capacity in rats by treated beta amyloid (25-35). AD is a major cause to form neurodegenerative disorder that is characterized by a cognition defection. The changes of pathology included neurofibrillary tangles associated with amyloid beta depositions, and other age-related.

Amyloid beta (Aβ or Abeta) is a peptide of 39-43 amino acids that appears to be the main constituent of amyloid plaques in the brains of Alzheimer's disease patients. The aggregates of Aβ is related to damage of neurons, and form of cognition defection. Aβ (25-35) is the major toxicity peptide of Aβ, the toxicity of Aβ (25-35) is similar or more to Aβ (1-40) or Aβ (1-42). Research on laboratory suggest that symptom of rats treated by by intracerebroventricular infusion (i.c.v.) of amyloid-beta (25-35) peptide is similar to clinical AD disease.

In the invention, the effect of dl-PHPB for improving short-term memory and space sentience learning capability was detected in rats treated by intracerebroventricular infusion (i.c.v.) of amyloid-beta (25-35) peptide. The results indicate that dl-PHPB significantly improves learning-memory, and space sentience learning capability of i.c.v. model in rats. In place navigation testing, the dl-PHPB significantly decreased the escape latencies of i.c.v. model in rats in dose dependent manner. The results suggested that dl-PHPB significantly improves learning-memory, space sentience learning capability of i.c.v. rats model in dose dependent manner. The speed of swimming was no difference among each group for tasting period. The results showed physical force of rats treated by intracerebroventricular infusion (i.c.v.) of amyloid-beta (25-35) peptide was not different, compared to control group. The experiments results indicated that dl-PHPB could prevent and treat aged dementia, in particular improved short-term memory capability of AD disease.

The platform of probe trial testing were investigated in Morris water maze test, in which the retain time of target quadrant, and time of first crossing the platform location was recorded when the platform was removed to detect the memory capability of rats for the platform. The results showed the rats treated with dl-PHPB significantly increased the retain time of target quadrant, and decreased the time of first crossing the platform location, compared to the mice treated with saline. The results indicate that dl-PHPB could improve spatial learning capability of i.c.v. rats model in dose dependent manner. The experiments results indicated that dl-PHPB could prevent and treat aged dementia, in particular improved spatial learning and memory capability of AD disease.

For clearing the mechanism of dl-PHPB for prevention and treatment of AD disease, the index related to oxidant stresses damage and cholinergic system were detected, in particular the activity of superoxide dismutase (SOD), level of MDA and activity of ChAT by measured in brain tissue in rats treated with dl-PHPB.

At 14 days after intracerebroventricular infusion (i.c.v.) of amyloid-beta (25-35) peptide, the activity of SOD significantly increased, compared to the control rats. But the activity of SOD significantly decreased in cortex of rats treated with dl-PHPB for 14 days, compared to the rats treated with saline. The results indicate that dl-PHPB could decreased the activity of SOD in cortex of i.c.v. rats model in dose dependent manner.

At 14 days after intracerebroventricular infusion (i.c.v.) of amyloid-beta (25-35) peptide, the level of MDA significantly increased, compared to the control rats. But the activity of SOD significantly decreased in cortex of rats treated with dl-PHPB for 14 days, compared to the rats treated with saline. The results indicate that dl-PHPB could decreased the level of MDA in cortex of i.c.v. rats model in dose dependent manner. The results indicated that dl-PHPB could improve disorder of the capacity of anti-oxygenation in brain, decrease production of lipid peroxidation, restore the balance of oxidation. dl-PHPB might be used to applicant for antioxidant and free radical scavenger in brain tissue.

The activity of ChAT no significantly changes in rats by intracerebroventricular infusion (i.c.v.) of amyloid-beta (25-35) peptide, compared to the control rats. But the activity of ChAT significantly increased in rats treated with dl-PHPB (39mg/kg) for 14 days, compared to the rats treated with saline. The results indicated that dl-PHPB might be used to applicant for application to enhance function of cholinergic neurons.

In the summary, dl-PHPB could significantly decrease the activity of SOD, and level of MDA in cortex of AD diseases at dose dependent manner. The results showed dl-PHPB could improve oxidative stress damage signifies to decrease lipid peroxides (MDA), and restore the balance of oxidation in brain tissue. dl-PHPB might improve neurons function signifies to enhance the activity of ChAT in AD disease.

In the invention, the effect of dl-PHPB for improving short-term memory, space sentience learning capability was detected in Samp8 mice. Senescence accelerated mouse (SAM) can be divided into two subtypes, namely R subtype and P subtype. Clinical feature of samP mice include deliplation, pachulosis, behavior disorder, survival period shortening, and so on. Clinical feature of samR mice is similar to normal mice, following normal aging progress. The type of samP mice included 12 subtype. Clinical feature of samP8 mice major include memory defect by aging progress and subregion pathology of CNS (such as cortex and hippocampus). As the reported, neurofibrillary tangles together with amyloid beta depositions and the changes of neurotransmitter were found in aging rat, including decrease of acetylcholine, increase of opioid peptides, y-aminobutyric acid and 5-hydroxytryptamine in cortex or hippocampus. Oxidative stress injury was found, following increase of lipid peroxide of brain, oxidation- antioxidation system disturbance and dysfunction of mitochondria. Conclusion SAMP8 may simulate senile dementia and its pathogenesis involves insufficient cortex mitochondrial function, decreased cholinergic nerve function, and oxidative stress.

In the invention, the effect of dl-PHPB for improving short-term memory, space sentience learning capability was detected in Samp8 mice by step down test. The results indicated that dl-PHPB could improve spatial learning and memory capability of SAMP8 mice. The experiments results indicated dl-PHPB could prevent and treat aged dementia, in particular improved spatial learning and memory capability of AD disease.

After step down testing, the results showed the rats treated with dl-PHPB demonstrated a significantly decreased the foot shock time and increased latent period, compared to the mice treated with saline. The experiments results indicated that dl-PHPB could enhance the ability of active and passive avoidance response, improve the ability of learning and memory. The results showed that dl-PHPB could prevent and treat aged dementia, in particular improved spatial learning and memory capability of mixed dementia disease. ,

The maze step through test was used to detect the ability of short-term memory and space sentience learning in mice. The escape latency time and number of errors in encountering blind ends were used to reflect the ability of learning and memory in mice. In the invention, the mice treated with dl-PHPB had the shorter escape latency time and the litter number of errors in encountering blind ends, compared to the mice treated with saline. The results indicate that dl-PHPB could improve short-term memory and spatial learning capability of SAMP8 mice in dose dependent manner. The results showed that dl-PHPB could prevent and treat aged dementia, in particular improved spatial learning and memory capability of mixed dementia disease. In order to elucidate the mechanism of dl-PHPB to prevent and treat aged dementia, in particular improving mixed dementia disease, the biochemical indexes related to aging and learning and memory were detected in brain by biochemical method, including activity of ChAT, AChE, SOD, and level of MDA. The results showed that in SAMP8 mice treated with dl-PHPB for 35 days, the activity of SOD (p<0.05) and level of MDA (p>0.05) in hippocampus, was decreased, compared to the mice treated with saline. The results indicated that dl-PHPB could improve disorder of the capacity of anti-oxygenation in brain, decrease production of lipid peroxidation, and restore the balance of oxidation.

ACh, one of the key neurotransmitters in the central nervous system, mediates the signaling pathway of cholinergic nerve and is closely involved in the learning and memory process. Choline acetyltransferase (ChAT) enzyme synthesizes the ACh and acetylcholinesterase (AChE) enzyme hydrolyzes the ACh, both activities of which indirectly reflect the ACh content and function of cholinergic nerve in the brain.

After successive application up to 35 days, hippocampal ChAT activity of SAMP8 mice in the dl-PHPB-treated groups has a statistically significant increase in comparison with control group and exhibited certain dose-dependency, which suggests dl-PHPB might decrease AChE activity in the hippocampus of SAMP8 mice.

So, dl-PHPB raises the ChAT activity in the hippocampus of mixed-dementia patient and lessens the AChE activity in the hippocampus with certain tendency, which implies that dl-PHPB might ameliorate the cholinergic function via increasing the ACh content in the hippocampus of mixed-dementia patient.

In a word, dl-PHPB has preventive, ameliorative and therapeutic effects on vascular dementia. Its multiple actions are as followed: (1) PHPB significantly improves the recent memory and spatial location memory impairment; (2) PHPB notably lessens the SOD activity compensatorily increasing in the brain of vascular dementia patients and level of lipid peroxidation product MDA, which suggests that PHPB might inhibit the insult on the neuron induced by oxygen stress; (3) PHPB increases the ChAT activity in the brain of vascular dementia patients possibly inducing the higher ACh level in favor of improving learning and memory; (4) PHPB improves the pathologic change in the brain of vascular dementia patients including sparse white matter, vacuolization, an increase in glial cells and abnormal neuron morphology, and ameliorates the decrease of Brain-derived neurotrophic factors induced by brain ischemia.

Meanwhile, PHPB has preventive, ameliorative and therapeutic effects on presenile dementia. Its multiple actions are as followed: (1) PHPB significantly improves the learning and memory of presenile dementia patient; (2) PHPB notably lessens the SOD activity compensatorily increasing in the brain of presenile dementia patient and level of lipid peroxidation product MDA, which suggests that PHPB might inhibit the insult on the neuron induced by oxygen stress and protect the neurons; (3) PHPB increases the ChAT activity further improving cholinergic function, which helps ameliorating learning and memory impairments in the presenile dementia patient.

In addition, PHPB has preventive, ameliorative and therapeutic effects on mixed dementia. Its multiple actions are as followed: (1) PHPB significantly improves the recent memory and spatial location memory impairment; (2) PHPB notably lessens the SOD activity compensatorily increasing in the brain of mixed dementia patient and level of lipid peroxidation product MDA, which suggests that PHPB might protect neurons from the insult induced by oxygen stress; (3) PHPB increases the ChAT activity and possibly decreased AChE activity in the hippocampus, which suggests that it might improve learning and memory impairments in the mixed dementia probably via boosting cholinergic functions.

In brief, PHPB has therapeutic effects on the dementia, and ameliorative effects on cognitive decline associated with aging process, which mechanisms are involved in reduction of insults induced by oxygen stress in the brain, enhancement of functions in the cholinergic nerve, increase of brain-derived growth factor.

On the other hand, the invention refers to pharmaceutical compositions for use in the prevention, amelioration or treatment of particular dementia-related signs, which include dl-PHPB at a prevention- or treatment-effective dose, as well as optional and pharmaceutical acceptable carriers and excipients. In the invention, the pharmaceutical compositions could be prepared as the following formulations on the basis of administration route: solution, suspension, emulsion, pill, capsule, powder, controlled-release or sustained-release preparation.

The administration routes, in which dl-PHPB compositions prepared with known methods are administered in the invention, included but are not limited to the following: parenteral, per os, focal, intracutaneous, intramusculary, intraperitoneally, subcutaneous, intranasal route. dl-PHPB in the invention could be prepared via the known method.

Optional dl-PHPB compositions in the invention could be prepared with one or multiple pharmaceutical acceptable carriers and /or excipients via any routine method. Therefore, dl-PHPB and its solvated forms could be specially prepared for inhalation, insufflations (via mouth or nose), per os, buccal, parenteral or rectum administration. dl-PHPB compositions could also take the solution, suspension, emulsion, pill, capsule, powder, controlled-release or sustained release formulation. These preparations contains dl-PHPB at treatment-effective dose optimized for purified form and an appropriate amount of carrier to provide patients proper administration options. The preparation should be consistent with administration route.

In the invention, the purified form of dl-PHPB as stated refers to basically pure dl-PHPB, especially with purity of more than 80%, optimized pure dl-PHPB with purity of more than 85%, specially optimized dl-PHPB with purity of more than 90%, even more optimized dl-PHPB with purity of more than 98%. On the whole, the purity of dl-PHPB as stated above ranges from 95% to 99%, for instance.

### Parenteral administration

dl-PHPB compositions could be prepared for parenteral administration via injection, for example, bolus infusion. The injection preparation lies in one ampoule as one unit formulation or multi-dose container with optionally additive preservatives. Parenteral formulation is placed into ampoules, disposable synringes or multi-dose containers made of glass or plastics et al. It could also take the form of suspension, solution or emulsion containing lipophilic or hydrophilic carriers and excipients, such as deflocculant, stabilizer and/or disperser.

For example, the parenteral preparation is one kind of sterile injection or suspension with non-toxic, extra- parenterally acceptable diluents or solvents (for example, solution dissolved in 1,3-butanediol). Acceptable carriers and available solvents include water, Ringer's solution and iso-osmotic sodium chloride solution. In addition, sterile and non-volatile oil is routinely used as solvents or suspension medium. Given this, any gentle and non-volatile oil could be used including synthetic monoglyceride and diglyceride. Moreover, fatty acids such as oleic acid are also employed in the parenteral preparation.

Besides, dl-PHPB compositions could also be prepared into powder form, which needed reconstruction with proper vehicle such as pyrogen-free and sterile water before administration. For example, dl-PHPB compositions suitable for parenteral administration included sterile and iso-osmotic solution, which contained 0.1% to 90% dl-PHPB by weight per volume. The content of PHPB in the solution is approximately 5% to 20%, optimized to approximately 5% to 17%, more optimized to approximately 8% to 14%, and even more optimized to 10%. The solution or powder formulation might contain solubilizer, and local anesthetic such as lidocaine to ease the pain in the injection site. It's known for the other parenteral administration method in this domain, which is included in the scope of this invention. Oral administration

dl-PHPB compositions could be prepared in the form of tablet or capsule via routine methods with pharmaceutical acceptable excipients such as adhesive, filler, lubricant and disintegrant.

### A. adhesive

The adhesives include but are not limited to maize starch, potato starch or other starch, gelatin, natural and synthetic gum such as arabic gum, algin, alginic acid, other alginate, powdered tragacanth, guar gum, cellulose and its derivatives (such as ethyl cellulose, cellulose acetate, calcium carboxymethylcellulose, sodium carboxymethyl cellulose), polyvinylpyrrolidone, methylcellulose, pregelatinized starch, cellulose hydroxypropyl methyl (such as, Nos. 2208, 2906, 2910), microcrystalline cellulose and its mixture. Proper format of microcrystalline cellulose, for instance, included the material sold as AVICEL-PH-101, AVICEL-PH-103 and AVICEL-PH-105 (from FMC Corporation, American Viscose Division, Avicel Sales, Marcus Hook, Pennsylvania, USA). An example of a suitable adhesive was the mixture of microcrystalline cellulose and sodium carboxymethyl cellulose sold as AVICEL RC-581 from FMC corporation.

### B. filler

The fillers include but are not limited to talc, lactose, microcrystalline cellulose, powdered cellulose, dextrates, kaolin, mannitol, silicic acid, sorbitol, starch, pregelatinized starch and its mixture.

### C. lubricant

The lubricants include but are not limited to calcium stearate, magnesium stearate, mineral oil, light mineral oil, glycerine, glucitol, mannitol, polyethylene glycol, other ethanediol, stearine, sodium lauryl sulfate, talc, hydrogenated vegetable oil (such as peanut oil, cotton oil, sunflower oil, sesame oil, olive oil, maize oil and bean oil), zinc stearate, aethylis oleas, Laurate ethyl, agar and its mixture. Other lubricants included, for example, solid silicone (AEROSIL 200, Baltimore, Maryland, USA, W.R. Grace Co.), condensation aerosol of synthetic silica (Degussa Co. of Plano, Texas, USA), CAB-O-SIL (a kind of pyrogenic silica product, sold by Cabot Co., in Boston, Massachusetts, USA) and its mixture.

### D. disintegrant

The disintegrants include but are not limited to agar-agar, alginic acid, calcium carbonate, microcrystalline cellulose, croscarmellose sodium, crospovidone, polacrilin potassium, sodium starch glycolate, potato or cassava starch, other starch, pregelatinized starch, clay soil, other algin, other cellulose, gum and its mixture.

Optional coating method in the art could be employed for tablets or capsules. If adhesives and/or fillers are used in the dl-PHPB composition, they are generally up to 50% to 99% by weight of the compound. On one hand, about 0.5% to 15% disintegrant by weight, especially about 1% to 15% disintegrant, could combine with dl-PHPB. Lubricant is optional and its content is no more than 1% on dl-PHPB by weight. The methods about the preparation of solid oral formulation and pharmaceutical acceptable additives are described in the Marshall, Solid Oral Dosage Forms, Modem Pharmaceutics (Banker and Rhodes, Eds.), 7: 359-427 (1979). Other less typical formulations are well known in the art.

The formulation of solution, syrup or suspension could be employed for oral liquid preparation. Or, liquid preparation could be in the form of dried product, and be reconstructed via water or suitable carrier before use. These liquid preparations are prepared with routine methods via pharmaceutical acceptable additives such as deflocculant (for instance, sorbitol syrup, cellulose derivatives or hydrogenated edible fat), emulsifier (for example, lecithin or acacia gum), hydrophobic carrier (for instance, apricot oil, oleaginous ester, alcohol, or fractionated vegetable oil), and/or preservatives (for example, methyi p-hydroxybenzoate, propyl p-hydroxybenzoate or sobic acid). Optional buffer salt, flavoring agent, colorant, aromatics and sweetening agent could be added into these formations. Oral formation might also be prepared into controlled-drug-release dosage form. Preferably, Oral dosage form contain from 10% to 95% compound. In addition, in the invention dl-PHPB compositions could also be prepared into buccal tablet or lozenge. Other PHPB oral administration routes are known to the skilled in the art and included within the invention.

### Controlled-release administration

Controlled (sustained)-release formation is designed to prolong the action time and decrease the administration frequency of PHPB. This kind of preparation could also influence the onset time or other properties such as compound level in the blood, thereby affecting the emerging of adverse effects.

The controlled-release formation is designed to initially release certain dl-PHPB attaining the therapeutically needed efficacy, then gradually and consecutively release additional dl-PHPB to maintaining therapeutic level in long course. In order to keep approximately constant compound level, dl-PHPB is released from the preparation at certain speed to replace the dl-PHPB metabolized and/or secreted in the body. Many induced factors stimulate the controlled release of dl-PHPB, such as change of pH, change of temperature, enzyme, water or other physiological conditions or molecules.

Controlled-release systems, such as delivery pump, could apply the compound in similar way of insulin or chemotherapy agent delivered to target organ or tumor. In the system, dl-PHPB usually combines with bio-degradable, bio-compatible polymer implants, which is characterized by dl-PHPB release at selected site in the control of time. Examples of polymeric materials includes polyanhydride, polyorthoesters, polyglycolicacid, polylacticacid, polyethylenevinylacetate and its copolymers and combinations. In addition, the controlled-release system is placed in the vicinity of the therapeutic target, resulting to only a fraction of whole-body dose needed.

In the invention, dl-PHPB could be applied via other controlled-release methods or drug delivery systems known to the skilled in the art, which includes hydroxypropyl methylcellulose, another polymermatrix, gel, permeable membrane, infiltration systems, multi-layer coating, particles, liposomes, microspheres, etc., or any combination of the above, with different mixing ratio to provide needed release spectrum. Other controlled-release methods of dl-PHPB are known to the skilled in the art and included within the invention.

### Inhalation administration

dl-PHPB could also be applied by inhalation administration via different devices conveniently delivering to patient's lung. For example, metered-dose inhaler ("MDI") contained suitable Low boiling point propellant in the tank, such as dichlorodifluoromethane, trichlorofluoromethane, dichlorotetrafluoroethane, carbon dioxide or other suitable gas, which directly deliver the compound to lung. MDI devices could be obtained from many suppliers, such as 3M Corporation, Aventis. Boehringer Ingelheim. Forest Laboratories, Glaxo-Wellcome, Schering Plough and Vectura.

In addition, dry powder inhaler (DPI) could also be employed to apply the compound. The DPI apparatus generally uses one mechanism, for example, the outbreak of the gas brings about cloud-form dry powder resulting in the inhalation by patients. The DPI apparatus is known in the art and available from many suppliers, such as Fisons, Glaxo-Wellcome, Inhale Therapeutic Systems, ML Laboratories, Qdose and Vectura. Multi-dose DPI ("MDDPI") system is its popular variable form permitting more than one therapeutic dose application. The MDDPI apparatus is available from many companies, such as AstraZeneca, GlaxoWellcome. IVAX, Schering Plough, SkyePharma and Vectura. For example, gelatin capsules and cartridges referring to inhaler and insufflators could be prepared into power composite containing PHPB and power matrixes suitable for this system, such as lactose and starch.

Another kind of apparatus referred to the application of compounds to the lung, such as liquid spray device provided by Aradigm Corporation. Liquid compound is atomized via a tiny nozzle in the liquid spray system, which is directly inhaled into the lung. For example, atomizer device could be employed for the application of compounds to the lung. Using ultrasonic energy, liquid compounds are transformed into the aerosol in the atomizer, which consists of small particles prone to the inhalation. Examples of atomizers, include the devices provided by Sheffield/Systemic Pulmonary Delivery Ltd, Aventis and Batelle Pulmonary Therapeutics.

In another embodiment, electro-hydrodynamic (EHD) aerosol device is used for the application of compounds to the lung. Liquid solution or suspension is atomized via energy in the EHD aerosol device. When the compound is applied to the lung through EHD aerosol device, the electrochemical properties on the compound preparation is the optimized important parameters. The optimization is routinely carried on by the skilled in the art. Other lung delivery methods on dl-PHPB are well known to the skilled in the art, and includes within the invention.

dl-PHPB preparation suitable for the atomizer, liquid spray device and EHD aerosol device, generally consists of dl-PHPB and pharmaceutically acceptable carriers. In a representative embodiment, the pharmaceutically acceptable carrier is a kind of fluid, such as alcohol, water, polyethylene glycol or perfluorocarbon. Optionally, the aerosol properties on the compound solution or suspension could be changed through the addition another substance. For example, the substance might be a kind of fluid, such as alcohol, diol, polyethylene glycol or fatty acids. Other preparative methods on the liquid compound solution or suspension suitable for the aerosol devices, are well known to the skilled in this art.

### Reservoir administration

dl-PHPB could also be prepared into reservoir preparation. Such prolonged action preparation is administered via implantation (such as subcutaneous or intramuscular) or intramuscular injection. Therefore, the compound could combine with suitable polymeric or hydrophobic materials, such as emulsion in the acceptable oil or ion exchange resin, or slightly soluble derivatives like slightly soluble salts. Other reservoir administration methods on the dl-PHPB are well known to the skilled in this art, and includes within the invention.

### Focal administration

For the focal administration, dl-PHPB could combine with the carriers so as to deliver effective dose. According to required activity, the effective doses ranges from 1.0 µM to 1.0 mM. In one aspect of the invention, focal administration of the compound composite could be applied to the skin. Carriers include, but not limited to the form of ointment, cream, gelatin, paste, foam, aerosol, suppository, pad or gel stick.

Focal preparation could comprise therapeutically effective compound in the ophthalmologically acceptable vehicles, such as buffed salt solution, mineral oil, vegetable oil like corn oil or peanut oil, vaseline, Miglyol 182, alcohol solution, liposome or liposome-like product. Any of these compounds could also comprise preservatives, antioxidants, antibiotics, immunosuppressant and other biologically or pharmaceutically effective agents with no harmful effects on the compound. Other focal administration methods on the dl-PHPB are well known to the skilled in this art, and included within the invention.

### Other delivery systems

Other delivery systems are well known to the skilled in the art, and could be used for the application of the compound in the invention. Moreover, these and other delivery systems could be combined or modified to optimize the dl-PHPB administration in the invention.

The inventive use can be realized in a method on the prevention, relief and/or treatment of the dementia disease or symptom, including therapeutically effective dose of dl-PHPB or drug composition containing dl-PHPB administered to patient in need. Administration routes include, but not limited to parenteral, per os, focal, intracutaneous, intramusculary, intraperitoneal, subcutaneous, intranasal route.

In the invention, said therapeutically effective dose of dl-PHPB is optionally from 0.5 to 200mg/kg body weight, optimized for 1-150mg/kg body weight, more preferred is 2-100mg/kg body weight, more preferred is 3-50mg/kg body weight, more preferred is 4-35mg/kg body weight, and more preferred is 5-20mg/kg body weight any dose between.

The determination principles of the therapeutically effective dose of dl-PHPB

In the invention, the term "the therapeutically effective dose" means that the subjects with the necessary therapeutically effective dose of PHPB were determined according to disease and extend of disease. For example, the dose of cure, prevent, inhibit or prevent or at least part of inhibit or prevent the target disease or condition.

The toxicity and efficacy of dl-PHPB was determined to LD50 (50% lethal dose groups) and the ED50 (50% effective dose groups) by the standard Pharmaceutical approach in cell cultures or experimental animals. Therapeutically index of dl-PHPB was ratio between toxicity and efficacy of a treatment dose, namely the ratio of LD50/ED50.

The data obtained from cell culture experiments and animal studies can be used in the preparation of humans and other mammals dose range used. dl-PHPB in the dose optimization with minimal toxicity or no toxicity, including ED50 of the concentration of circulating plasma or other body fluids.

The dose range of dl-PHPB depends on the dosage form and route of administration. In the invention, the effective dose of dl-PHPB was estimated according to dose of animal experiments. The dose of dl-PHPB was designed to achieve IC50 of plasma concentration in animal models. Then, the effective dose of dl-PHPB could be more accurately determined according to the information in the humans and other mammals. The level of dl-PHPB in plasma could be measured by high performance liquid chromatography.

The effective dose of a single dosage dl-PHPB combination with pharmaceutical acceptable carriers was determined according to the host and the specific and different delivery modes. The field technicians should understand that the content of dl-PHPB in individual dose of each formulation does not require achieving the effective dose by itself, because it was easy to achieve the required dose by applying to multi-individual dose of each formulation. The dose of dl-PHPB chosen depend on formulations, disease, and the specific purpose to be decided by the field technicians

The dose programs of dl-PHPB used to treat diseases was chosen to whether apply delivery system of dl-PHPB, according to multi-factors: including the type of patient, age, weight, sex, diet, medical condition, route of administration, and pharmacological factors such as activity, efficacy, the characteristic of pharmacokinetics and the distribution of toxicology. Therefore, the actual dose program of dl-PHPB may be very different from subject to subject. Terms and abbreviations
dl-PHPB, PHPB is dl-2 - (1-hydroxy-pentyl) benzoic acid potassium salt
SOD is superoxide dismutase
MDA, is the malondialdehyde,
ChAT is the choline acetyltransferase,
AChE is acetylcholinesterase,
ATPase is the triphosphate phosphohydrolase.

### Figure Legends

Fig.1. Effects of dl-PHPB on the escape latencies of permanent 2-VO rats in water maze performance. Values are meantS.E.M.(N=17-20). ^{#}P<0,05, ^{# #}P<0.01v.s. sham group; *P<0.05 v.s.vehicle group (LSD test).
Fig.2. The typical swimming-tracking paths of permanent 2-VO rats in Morris water maze. A: marginal mode; B: random mode; C: tendency mode; D: linear mode.
Fig.3. Effects of dl-PHPB on the time in the platform-quadrant (A) and the first crossing-platform time (B) of permanent 2-VO rats in Morris water maze after administering for 21 days. Values are mean±S.E.M.(N=17-20). *P<0.05, ***P<0.01 v.s.vehicle group (LSD test).
Fig.4. Effects of dl-PHPB on the biochemical indexes of brain tissues from permanent 2-VO rats. A: activity of SOD in cortex; B: level of MDA in cortex; C: activity of ChAT in hippocampus. Values are mean±S.E.M.(N=5-8). ^{###}P<0.001 v.s. sham group; *P<0.05, **P<0.01,***P<0.001 v.s.vehicle group (LSD test).
Fig.5. Effects of dl-PHPB on photomicrographs of hematoxylin and eosin staining of cerebral cortexes of permanent 2-VO rats after administering for 21 days. Magnification, 400 X
Fig.6. Effects of dl-PHPB on photomicrographs of hematoxylin and eosin staining of hippocampus CA1 region of permanent 2-VO rats after administering for 21 days. Magnification, 400 X
Fig.7. Effects of dl-PHPB on photomicrographs of hematoxylin and eosin staining of hippocampus CA3 region of permanent 2-VO rats after administering for 21 days. Magnification, 400 X
Fig.8. Effects of dl-PHPB on photomicrographs of klüver-barrera luxol fast blue staining of corpus callosums of permanent 2-VO rats after administering for 21days. Magnification, 400 X
Fig.9. Effects of dl-PHPB on photomicrographs of klüver-barrera luxol fast blue staining of optic tracts of rats after administering for 21days. Magnification, 400 X
Fig.10. Effects of dl-PHPB on photomicrographs of the immunohistochemical staining for GFAP in the hippocampus of permanent 2-VO rats after administering for 21days. Magnification, 200 X
Fig.11. Effects of dl-PHPB on photomicrographs of the immunohistochemical staining for GFAP in the optic tracts of permanent 2-VO rats after administering for 21_days. Magnification, 200 X
Fig.12. Effects of dl-PHPB on active astrocytes in brain of permanent 2-VO rats after administering for 21days.A: cortex, B: hippocampus, C: corpus callosum, D: optic tract. Values are mean±S.E.M.(N=4). ^{# #}P<0.01 v.s. sham group; *P<0.05, **P<0.01 v.s.vehicle group (LSD test).
Fig.13. Effects of dl-PHPB on the area and density of BDNF in cortex of permanent 2-VO rats after administering for 21days. Magnification, 200 X
Fig.14. Effects of dl-PHPB on photomicrographs of the immunohistochemical staining for distribution and content of GFAP in the hippocampal CA1 area of permanent 2-VO rats after administering for 21days. Magnification, 200 X. A: sham group; B: vehicle group; C: PHPB 39mg/kg group.
Fig.15. Effects of dl-PHPB on photomicrographs of the immunohistochemical staining for distribution and content of GFAP in the hippocampal CA2 area of permanent 2-VO rats after administering for 21days. Magnification, 200 X. A: sham group; B: vehicle group; C: PHPB 39mg/kg group.
Fig.16. Effects of dl-PHPB on photomicrographs of the immunohistochemical staining for distribution and content of GFAP in the hippocampal CA3 area of permanent 2-VO rats after administering for 21days. Magnification, 200 X. A: sham group; B: vehicle group; C: PHPB 39mg/kg group.
Fig.17. Effects of dl-PHPB on the content of BDNF in brain tissue of permanent 2-VO rats after administering for 21days. Values are mean±S.E.M.(N=4). ^{#}P<0.05, v.s. sham group; *P<0.05, **P<0.01v.s.vehicle group (LSD test).
Fig.18. Effects of dl-PHPB on the escape latencies of Aβ(25-35)-induced dementia rats in Morris water maze performance after administration. Values are mean±S.E.M.(N=7-9). ^{#}P<0.05, v.s. sham group; *P<0.05, **P<0.01v.s.vehicle group (LSD test).
Fig.19. Effects of dl-PHPB on the time in the target-quadrant and the first crossing-platform time of Aβ(25-35)-induced dementia rats in Morris water maze performance after administration. A: dl-PHPB increased dose-dependently the time in the target-quandrant of dementia rats compared with the vehicle rats; B: dl-PHPB had a tendancy of reducing the first crossing-platform time. Values are means±S.E.M.(n=7-9). #P<0.05 vs. sham group; *P<0.05 vs. vehicle group (LSD test).
Fig.20. Effects of dl-PHPB on the biochemical indexes of Aβ(25-35)-induced dementia rats after administering for two weeks. A: cortex SOD activity; B: cortex MDA level; C: the cortex ChAT activity. Values are means±S.E.M.(n=7-9). ^{#}P<0.05 vs. sham group; *P<0.05, **P<0.01 vs. vehicle group (Dunnett or LSD test).
Fig.21.Effects of dl-PHPB on the shock number and latency of eleven-month-old SAMP8 in step down test after administering for 30 days. A: dl-PHPB reduced dose-dependently the shock number suffered by SAMP8; B: dl-PHPB increased dose-dependently the shock latency of SAMP8. Values are means±S.E.M.(n=11-14). *P<0.05, **P<0.01, ***P<0.001 vs. control group (Dunnett test).
Fig.22. Effects of dl-PHPB on the numbers of SAMP8 entering non-exit and the latencies of SAMP8 finding the steps in water maze after administering for 32-35 days. A: number of SAMP8 entering non-exit; B: latency of SAMP8 finding the steps. Values are means±S.E.M.(n=11-14). *P<0.05, **P<0.01 vs. control group (Dunnett test).
Fig.23. Effects of dl-PHPB on the SOD activity and MDA level of brain tissues from SAMP8. A: hippocampus SOD activity; B: hippocampus MDA level. Values are means± S.E.M. (n=11-14). ^{#}P<0.05 vs. sham group;( LSD test).
Fig.24. Effects of dl-PHPB on the ChAT and AChE activity of brain tissues from SAMP8. A: hippocampus ChAT activity; B: hippocampus AChE activity; C: cortex ATPase activity of Mitochondria. Values are means± S.E.M. (n=11-14). ^{#}P<0.05 vs. sham group;( LSD test).
Fig.25. The experiment design and schedule of example1.
Fig.26. The experiment design and schedule of example 2.
Fig.27. The experiment design and schedule of example 3.

The specific implementations

EXAMPLE 1: The study of chronic cerebral ischemia in rat: dl-PHPB improve the short-term memory and spatial learning capability of chronic cerebral ischemic rat.

### 1.MATERIALS AND METHODS

dl-PHPB was offered by the department of medical synthetic chemistry of our institute with a purity of more than 98.5%. dl-PHPB was dissolved in distilled water. Piracetam tablets were purchased by Tianjin Jinshi Pharmaceutical limited company. The levels of SOD, ChAT, and MDA activities were determined with commercial colorimetric assay kits (Nanjing Jiancheng Bioengineering Institute, China). The content of protein in the supernatant was determined by Bradford method using BSA as standard. Neutral red and fastness blue dye purchased by Sigma. Lithium carbonate purchased by Beijing Chemical reagent limited company. Triton X-100 purchased by zhongshan goldenbridge biotechnology co., LTD. Antibody of GFAP purchased by_ Chemicon. Antibody of BDNF purchased by Santa Cruz Biotechnology co., LTD. Other reagents purchased by zhongshan goldenbridge biotechnology co., LTD.

### 2.Instrument

The water maze apparatus was designed by Institute of Material Medica, Chinese Academy of Medical Sciences. Enzyme mark instrument (MQX 200) was purchased by Bio Tek Instruments. Paraffin section machine (IR2135) purchased by German Leica co., LTD. Thermostatic freezing section machine (620-E) purchased by UK Shandon co., LTD. Automatic microphotography system (Nikon ECLIPSE 80i) purchased by Japanese Nikon Corporation.

### 3. Preparation of 2-VO rat model

Chronic cerebral hypoperfusion was created by a permanent, bilateral occlusion of the common carotid arteries (2-VO) of adult rats. The rats were anesthetized with 10% trichloro-acetaldehyde, and temperature probe was inserted into the rectum, and a separate heating lamp was used to maintain rectal temperature at normothermic level. After a midline neck skin incision, the common carotid artery were exposed and ligated by a 5-0 nylon suture.

### 4. Treatment Groups and Drug Administration.

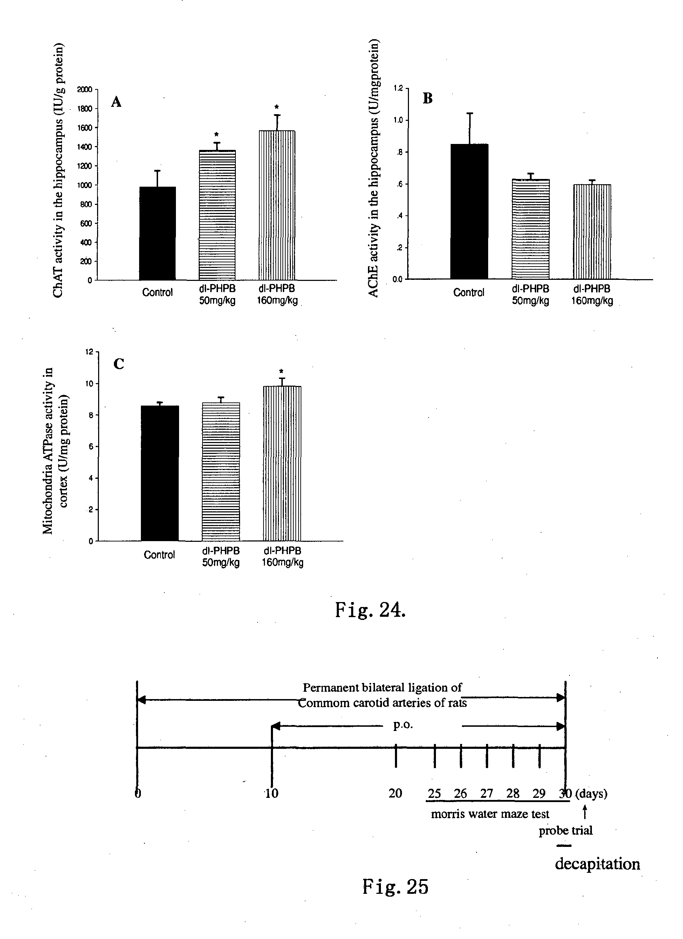

Rats were divided into six experimental groups randomly (20rats/each group) : one sham-operated group, one vehicle control group, one piracetam-treated group (600mg/kg), and three dl-PHPB-treated groups(13, 39, and 129 mg/kg). At 10 days after onset of 2-VO, drugs and vehicle were administered orally to rats for 21 days (one time /each day). The spatial learning and memory capability of rats were detected by Morris water maze in the 25-30days after operation. The testing of Morris water was commenced at 40min after drug treatment. The biochemical and pathology assays conducted later (in 24 h) after behavioral testing. The experimental schedule is shown in Fig. 25.

### 5. Morris water maze

Subjects were tested in a Morris water maze. This test is based on a standardized assessment of a spatial learning ability. The water maze apparatus consisted of a circular, stainless pool (120 cm in diameter, 60 cm in height). It was placed in a dimly lit, sound-proof test room. Multiple distant cues around the room (window, cabinets, furniture) were kept in the same location throughout the experiments. The water was filled to a depth of 40 cm at 25±1°C and was made opaque by adding milk powder to prevent visualization of the platform. A transparent platform (10 cm diameter) was put 1.5 cm below the surface of the water. The tank was divided into four quadrants with the platform in a fixed position in one quadrant. The subjects were placed into the maze facing the pool wall and were allowed two trials per day, 60 s per trial, to find the hidden platform. If the subject found the platform within the 60 s, it was given a 10-s rest period on the platform between trials. If the pedestal was not located within the time allotted, the subject was placed on to the platform and allowed 10 s until the next trial. The escape latency (time to reach the platform) was used to assess acquisition of the water maze task. Sessions were repeated for five consecutive days. On the sixth day, the platform was removed and the rat was allowed to search for the platform for 60 s (probe test). The time in the platform quadrant and latency time to cross platform location were recorded to measure the spatial learning ability without the influence of chance encounters with the platform. The Morris water maze sessions were recorded with a video camera for offline analyzing.

### 6. Biochemical assays

The biochemical assays conducted later (in 24 h) after behavioral testing. Eight rats in each group were anesthetized with ether and sacrificed. The brains were quickly removed and cleaned with ice-cold saline. Then the parietal cortex and hippocampus were isolated. For biochemical analysis, the tissues were weighed and homogenized in a proportion of 1:9 (w/v) in ice-cold saline. Homogenization (IKA, Germany) was carried out for 2 min in an ice bath. After the homogenates were centrifuged at 2,000×g for 10 min at 4°C (Sigma, Germany), the supernatant was used for analytical procedures. The levels of choline acetyltransferase (ChAT) SOD, and MDA activities were determined with commercial colorimetric assay kits (Nanjing Jiancheng Bioengineering Institute, China). The content of protein in the supernatant was determined by Bradford method using BSA as standard.

### 7. Histopathology and immunohistochemistry

### Hematoxylin-eosin and Klüver-Barrera staining

Four rats in each group were anesthetized with sodium pentobarbital (100 mg/kg, intraperitoneal injection), and perfused transcardially with cold saline, followed by 300 ml of 4% paraformaldehyde in 0.01 M phosphate buffer (pH 7.4). After these procedures, the brains were removed and stored in the 0.01 M phosphate buffer (pH 7.4) with 4% paraformaldehyde and 30% sucrose at 4°C until fully equilibrated. Then they were postfixed at 4°C, dehydrated and embedded in paraffin blocks. Coronal sections of 8 µm were stained with hematoxylin-eosin and Klüver-Barrera.

### GFAP and BDNF immunolabeling

The method for tissue fixation was described above. Serial coronal sections (40 µm) of the brains were cut through the dorsal hippocampus on a freezing microtome and collected in 6-well plates containing 0.01 M PBS. The detection of GFAP and BDNF immunoreactivity was performed using a conventional avidin-biotin-immunoperoxidease technique.

For quantitative analysis, one from every five samples in a continuous series of hippocampal or cortical tissue sections was taken, and was processed immunohistochemically. Thus, three slides were taken from each rat, and were read under an objective (× 1.6) microscope. The total area of positively stained neurons was counted with Image-pro Plus 5.0 software.

### 8. Statistical analysis

The results were expressed as mean±SEM. The data from training trail in the Morris water maze were analyzed by two-way analysis of variance (ANOVA) to detect the difference between groups and over time. The post hoc Dunnett's test was used to test the differences between two groups. Probe trial, biochemical assay, and immunohistochemistry assay were statically analyzed using one-way ANOVA followed by post hoc Dunnett's test. The results were considered to show a significant difference when the p value was less than 0.05.

### Results

### 1. dl-PHPB significantly improved chronic cerebral hypoperfusion-induced impairments in spatial learning and spatial working memory in rats.

Rats were given dl-PHPB daily for 21 days at 10 day after operation. And they were tested in the Morris water maze in the 25-30days after operation. The escape latency was used to reflect an aspect of cognition and spatial learning. Two-way ANOVA with repeated measures revealed a significant day effect on escape latency within groups, indicating that all group of rats improved their performance over the 5-day training period. Also, we found a significant treatment effect on escape latency.

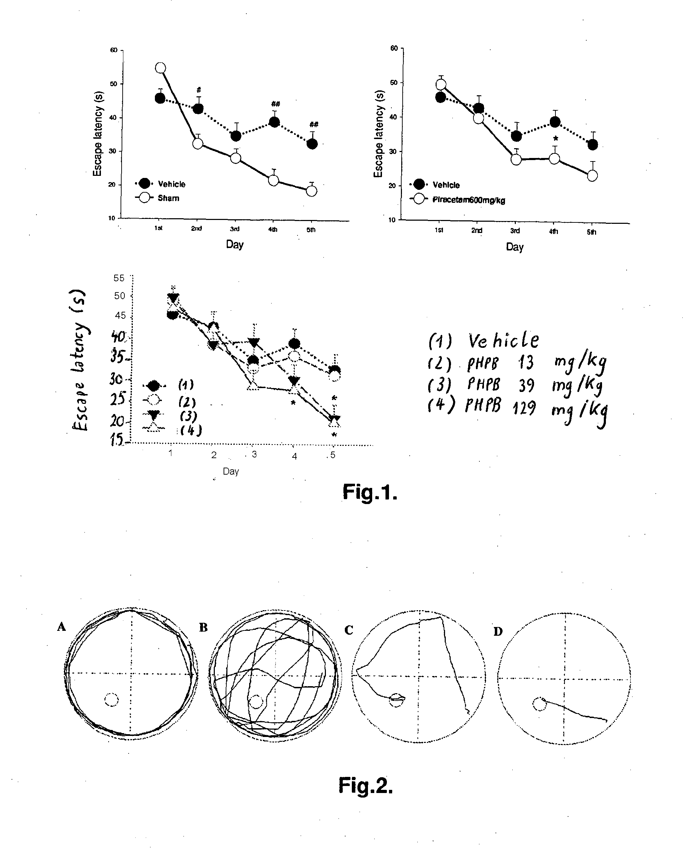

Post hoc analysis confirmed the internal validity of the study. 2-VO rats showed a significant longer time to find the platform (escape latency) than sham-operated rats (p<0.01). dl-PHPB treated rats performed better the fifth day (as indicated by statistical analysis), whereas sham operated rats performed better from the second day. The results suggested that chronic cerebral hypoperfusion successfully induced a learning deficit. Second, we found that 13mg/kg dl-PHPB-treated rats did not differ from the vehicle control animals. These results indicate that daily administration of 39 and 129mg/kg dl-PHPB significantly rescued learning impairment caused by Chronic cerebral hypoperfusion in escape latency in the water maze task (Fig. 2, Tab. 1 and 2.).

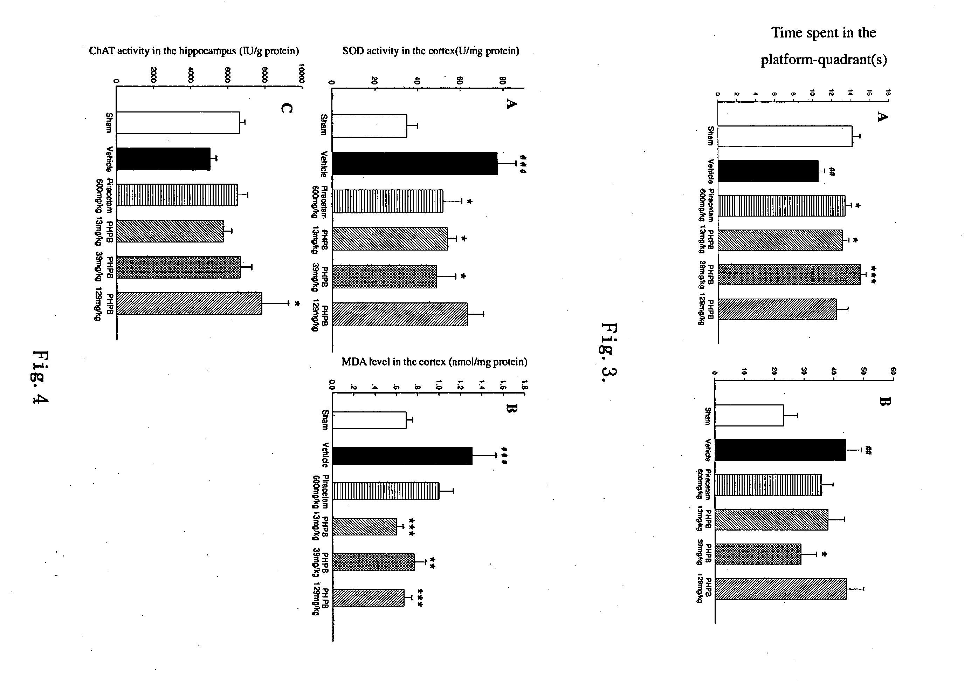

In probe trial day when the platform was moved, rats that have learned the location of the hidden platform are expected to spend the majority of the trial searching for the platform in the target quadrant.2-VO rats spent less time in the platform quadrant (p<0.01) and took more time to cross the platform location (p<0.01) than sham-operated animals. dl-PHPB (13_and 39 mg/kg) markedly showed ameliorative effect compared to 2-VO animals.(Fig.3A-B)

These results suggested that 2-VO rats underwent spatial cognition impairments, and dl-PHPB attenuated the learning and memory damages in 2-VO rats.

### 2. Effects of dl-PHPB on the SOD, ChAT activity and MDA level of brain tissues from permanent BCCAO rats.

SOD play important roles in maintenance the balance of oxidation, antioxidant with radical scavenging action. However, MDA is a major peroxide. The activity of SOD and the level of MDA reflected the level of antioxidation in brain tissue. After behavioral testing, the rats were sacrificed and the activities of ChAT, SOD, and MDA in the cortex and hippocampus were measured. The results are shown in Fig.4. In the cortex of 2-VO rats, SOD activity (77.39 ± 8.70 U/mg protein) was markedly higher than sham-operation group(35.03 ± 5.20 U/mg protein, P<0,001), and MDA level was increased from 0.69 ± 0.06 nmol/mg protein in sham-operation group to 1.31 ± 0.22 nmol/mg protein in 2-VO rats (p<0.001). dl-PHPB at 13 and 39mg/kg and piracetam at 600mg/kg significantly alleviated the increase of SOD activity in 2-VO rats. Meanwhile, dl-PHPB treatment decreased MDA level at the dose of 13, 39, and 129mg/kg in cortex of 2-VO rats. However, piracetam at 600mg/kg did not show significant effects on MDA in cortex of 2-VO rats. Furthermore, no significant effects of dl-PHPB and piracetam on the SOD activity and MDA level were observed in the hippocampus of 2-VO rats.

Acetylcholine is a neurotransmitter in the central nervous system, mediated by cholinergic nerve signaling. Acetylcholine is closely related with learning and memory, and synthesis by ChAT. So, the activity of ChAT can indirectly reflect levels of acetylcholine and reflecting the status of cholinergic function. Rats with permanent occlusion of bilateral common carotid arteries for 1 month, ChAT activity decreased significantly (a decrease of 24%) in the hippocampus of 2-VO rats, compared with sham operation group. After administration of 21 days, dl-PHPB 129mg/kg significantly increased the activity of ChAT in hippocampus (P <0.05). However, dl-PHPB at 13, 39mg/kg and piracetam at 600mg/kg did not show significant effects on activity of ChAT in hippocampus of 2-VO rats (Figure 4C). In the cortex, dl-PHPB and piracetam had no effect on ChAT activity (data not shown).

In conclusion, dl-PHPB can reduce SOD activity, and also decrease level of MDA in the cortex of 2-VO rats. The results indicated that dl-PHPB could improve disorder of the capacity of anti-oxygenation in brain, decrease production of lipid peroxidation, and restore the balance of oxidation. In addition, dl-PHPB maybe improves ChAT activity and cholinergic function in 2-VO rats.

### 3. Effects of dl-PHPB on the neuronal morphology in 2-VO rats

### (1) HE staining

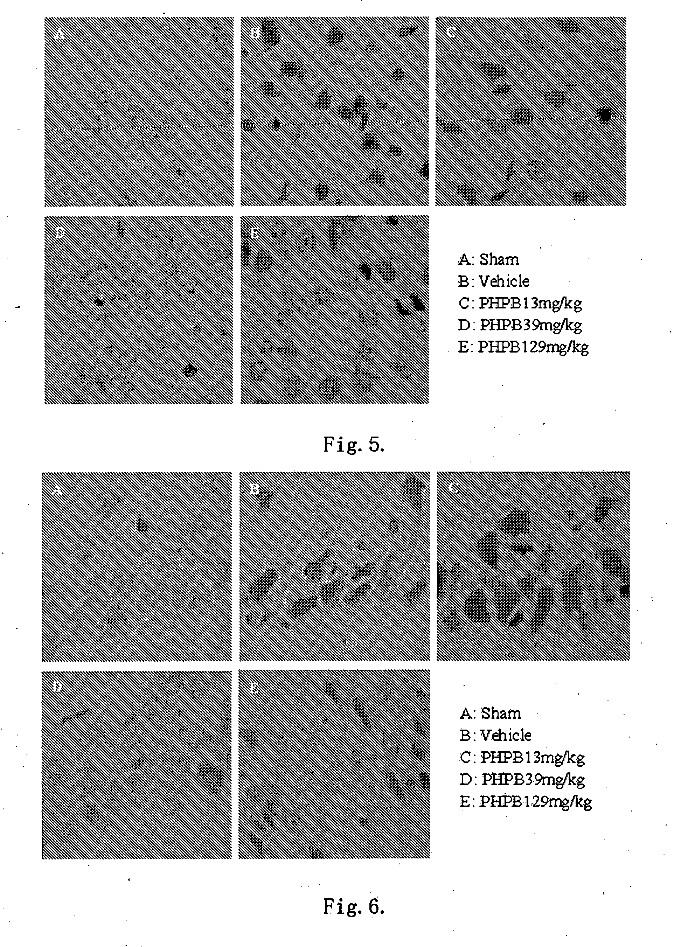

With HE staining in the cytoplasm and nucleus in different colors, one can clearly see the general shape of cells. Sham operation group in this study shows that cortical neurons shrink, deeply stained, nuclear is not clear, hippocampal CA1, CA3 areas also appear similar to the changes, but to a lesser extent, the hippocampus CA2 area were not affected. dl-PHPB 39mg/kg could significantly improve the cortex, hippocampus area CA1 and CA3 neurons form. dl-PHPB 129 mg/kg could improve the cortex and hippocampus CA1 area neuronal morphologic abnormalities, and dl-PHPB 13mg/kg only resulted in minor improvements of the abnormal shape of cortical neurons (Figure 5-7). Above that, dl-PHPB could protect the damage of cortex and hippocampus neurons caused by chronic cerebral hypoperfusion.

### 2) K-B staining

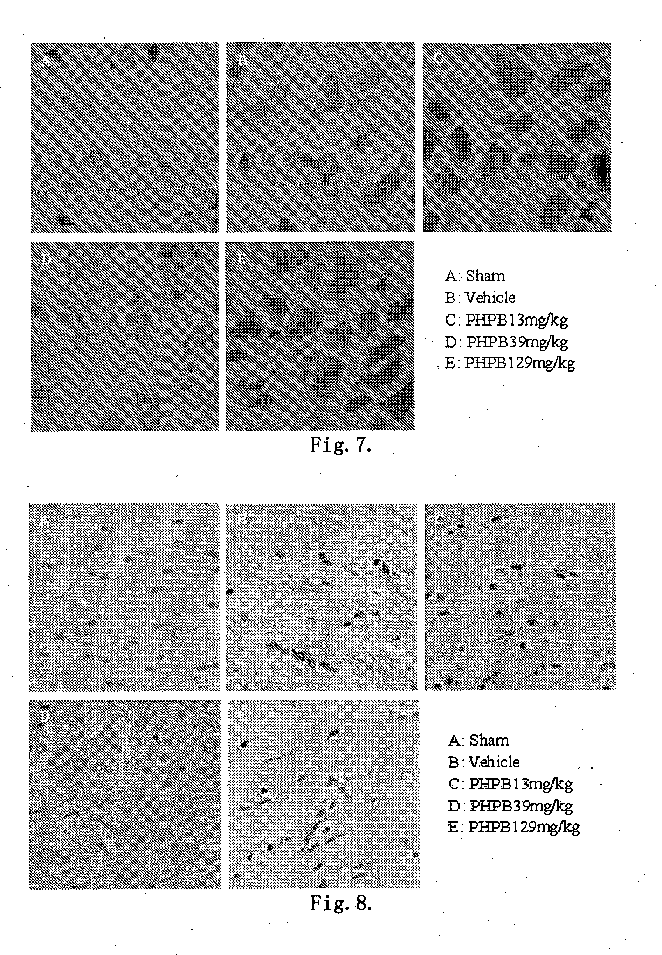

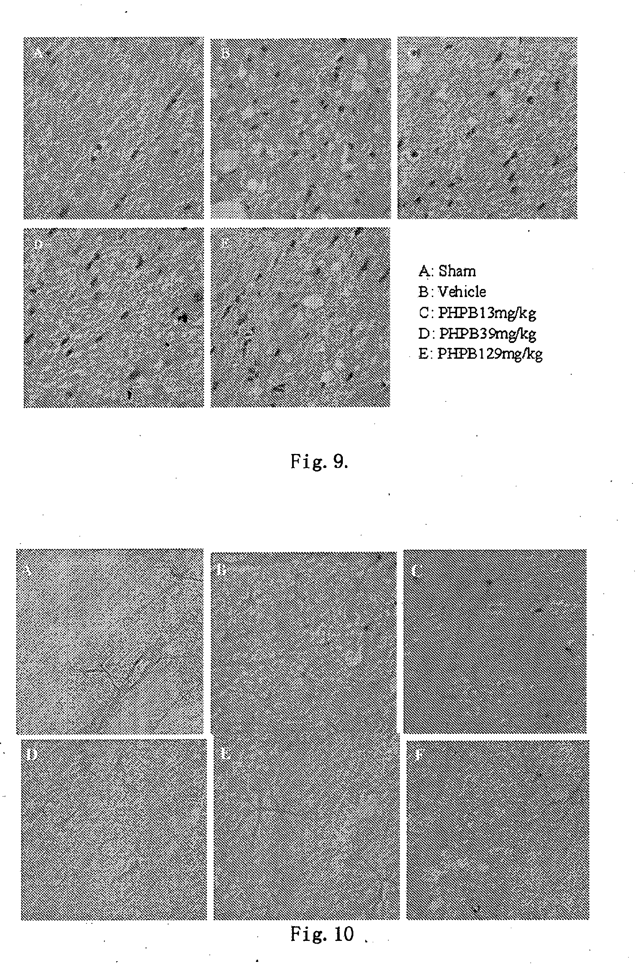

KB staining could reflect the morphological changes of neuronal myelin sheath, reflecting the morphological changes of nerve fibers. It was found that the corpus callosum and optic tract show clear vacuolization and nerve fiber disorders in sham operation group. dl-PHPB significantly improved the pathological damage of the corpus callosum, reduced vacuolization and restored nerve fiber arrangement, including dl-PHPB 39mg/kg strongest, dl-PHPB 129-mg/kg followed, dl-PHPB 13mg/kg the weakest (Figure 8.) dl-PHPB also has a certain improvement in the pathological changes of the optic tract, which the strong role of dl-PHPB 39mg/kg (Figure 9). These results suggest that, dl-PHPB could significant protect injury of the corpus callosum and optic tract in 2-VO rats.

### 4. Effects of dl-PHPB on the expression of GFAP-positive astrocytes in 2-VO rats

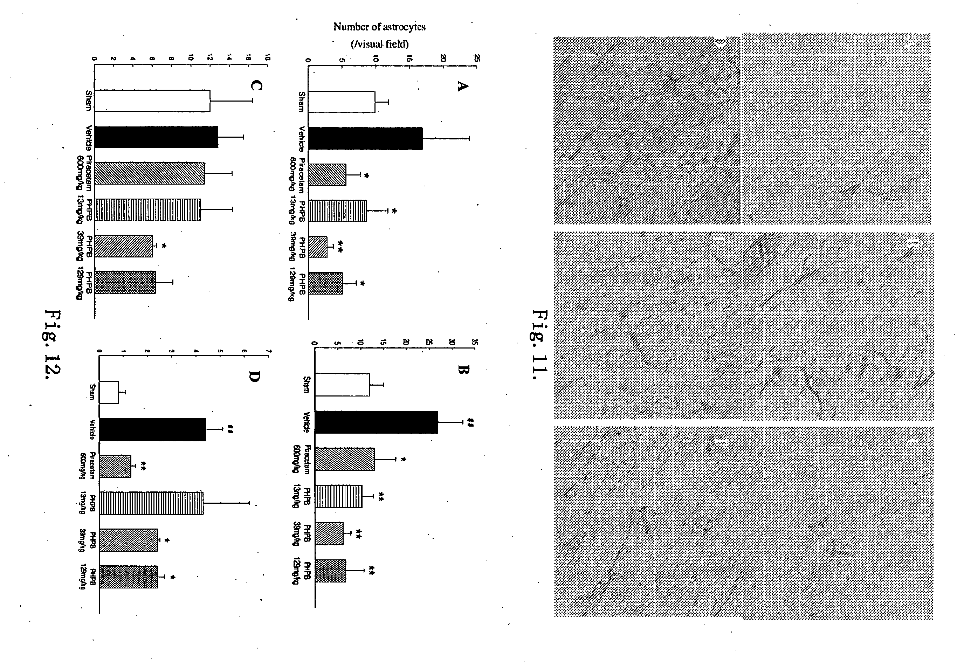

Cerebral damage caused by hypoperfusion may be the basis for spatial learning and memory impairment. In which the earliest white matter damage, accompanied by an increase of astrocytes and microglia activation. Glial fibrillary acidic protein (GFAP) immunohistochemical staining can be used to label activated astrocytes. Each selected slices in the same regions, each take 2-3 photos, record fixed-size view each photo in the number of astrocytes was calculated as the average value of the region. Four regions were selected to observe: the cortex, hippocampus, corpus callosum and optic tract. The results showed that the GFAP positive cells increased in the cortex of 2-VO rats (16.9± 6.9), but not statistically significant. After dl-PHPB and Piracetam administration for 21 days, GFAP-positive cells were significantly less than the vehicle group, in particular, dl-PHPB 39mg/kg has the most significant effect (P <0.01) (-Figure 12A). In the hippocampus, GFAP positive cells significant increased in 2-VO rats (26.8±5.5), compared with sham operation group (12.0±3.0, P<0.01). While dl-PHPB and Piracetam after administration for 21 days, GFAP-positive cells were significantly less than the vehicle group (P <0.05 or P <0.01), in particular, dl-PHPB39_mg/kg, 129 mg-/-kg has the most significant effect (P <0.01) (Figures 10 and 12B). In the corpus callosum, there are no significant differences between sham operation group and vehicle group, but the dl-PHPB 39mg/kg significant decreased level of GFAP positive cells (P <0.05) (Figure 12C). In the optic tract, GFAP positive cells significantly increased in 2-VO rats (4.4 ± 0.7), compared with sham operation group (0.8 ± 0.3, P <0.01). After dl-PHPB 39 mg/kg, 129 mg-/-kg, and Piracetam administration for 21 days, GFAP-positive cells were significantly less than the vehicle group (P <0.05 or P <0.01) (Figure 11 and 12D).

Described above, dl-PHPB can significantly improved cerebral damage of 2-VO rats and reduced the number of activated astrocytes, particularly in the hippocampus, optic tract and cortex, and dl-PHPB 39_mg/kg strongest, dl-PHPB 129_mg/kg followed, dl-PHPB 13_ mg/kg weakest.

### 5. Effects of dl-PHPB on the expression of BDNF in 2-VO rats

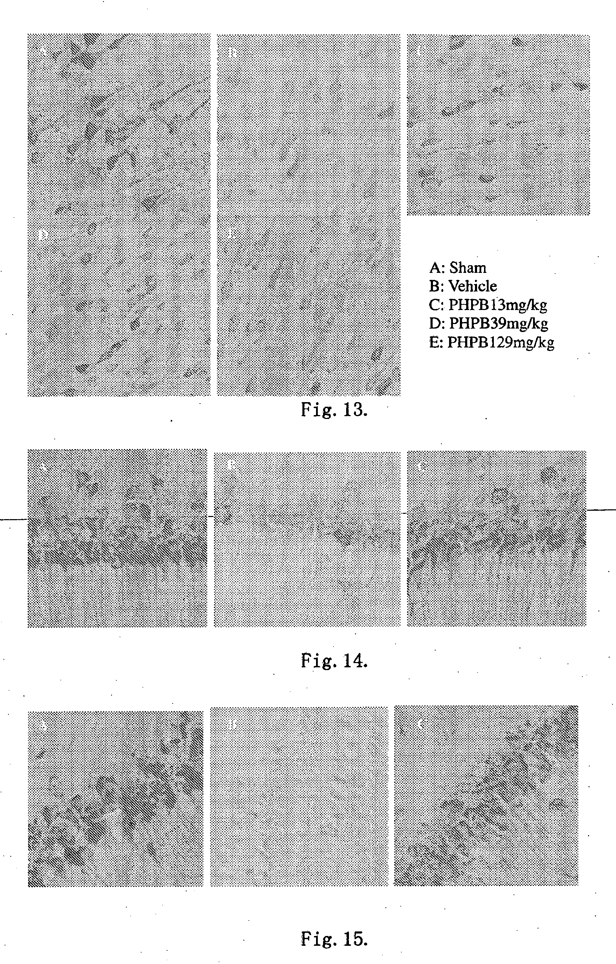

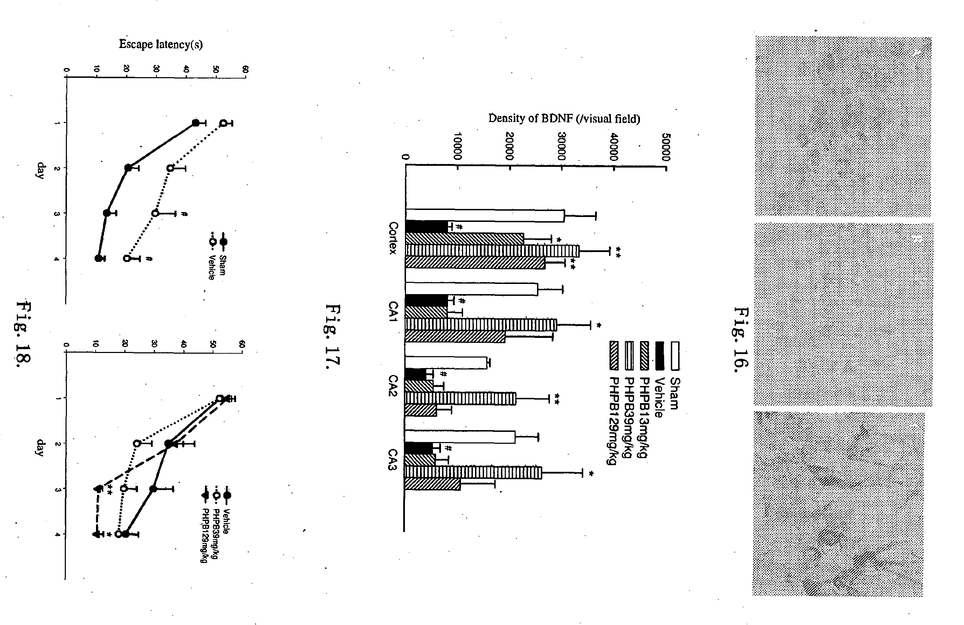

Brain-derived neurotrophic factor (BDNF) can maintain the survival and development of neurons, present in normal brain tissue of animals. In the cortex and hippocampus, there are no significant differences in treatment groups and vehicle group based on the area of BDNF immunohistochemistry (data not shown). However, based on staining intensity, expression of BDNF was significantly reduced both in the cortex or hippocampus of 2-VO rats, compared with sham group. In the cortex, dl-PHPB could increase the expression of BDNF, in which dl-PHPB 39_mg/kg strongest, dl-PHPB129_ mg/kg followed, dl-PHPB 13_mg/kg the weakest (Figure 13 and 17). In the hippocampus CA1, CA2, CA3 area, dl-PHPB 39mg/kg significantly increased BDNF expression (P <0.05 or P <0.01), dl-PHPB 129_mg/kg increased BDNF (only a trend which was not statistically significant, Figure 14-17). As the dye is proportional to density and BDNF levels, while the staining area within a certain range does not take into account the depth of staining, the staining density more accurately than the stained area reflects the content of BDNF. These results suggest that dl-PHPB could increase levels of BDNF in brain tissue of 2-VO rats.

### EXAMPLE 2: The study of Aβ(25-35)-induced dementia in rat: dl-PHPB improves the memory and spatial learning capability of Aβ(25-35)-induced dementia in rat.

### 1. Materials and methods

dl-PHPB was offered by the department of medical synthetic chemistry of our institute. dl-PHPB was dissolved in PBS. Aβ(25-35) was purchased by SIGMA. The levels of SOD, ChAT, and MDA activities were determined with commercial colorimetric assay kits (Nanjing Jiancheng Bioengineering Institute, China). The content of protein in the supernatant was determined by Bradford method using BSA as standard.

### 2. Instrument

The water maze apparatus was designed by Institute of Material Medica, Chinese Academy of Medical Sciences. Enzyme mark instrument (MQX 200) was purchased by Bio Tek Instruments. Paraffin section machine (IR2135) purchased by German Leica co., LTD. Thermostatic freezing section machine (620-E) purchased by UK Shandon co., LTD. Automatic microphotography system (Nikon ECLIPSE 80i) purchased by Japanese Nikon Corportion.

### 3. Preparation of Aβ(25-35)-induced dementia in rat

Aβ(25-35)-induced dementia model in 10 months of Male Wistar rats with 600g B.W. was created by slow injection of 15_nmol (volume of 5µl) Aggregation of Aβ (25-35). Sham-operation group was only injected by PBS (volume of 5µl). After operation, the animals were injected penicillin 200 000 units for 4 days by intraperitoneal injection.

### 4. Treatment Groups and Drug Administration.

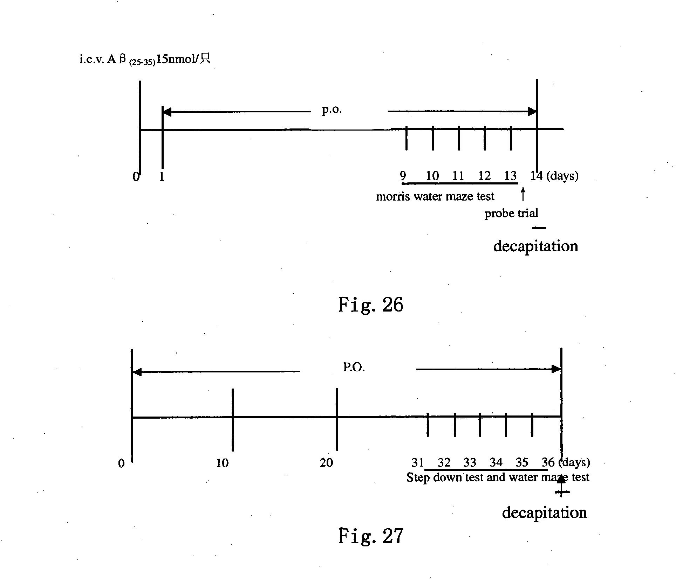

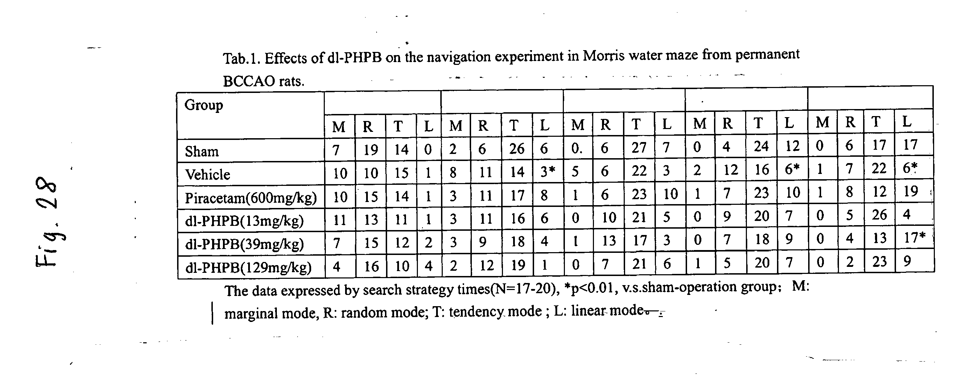

Rats were divided into four experimental groups randomly (10rats/each group) : one sham-operated group, one vehicle control group, and two dl-PHPB-treated groups_(39 mg/kg and 129 mg/kg). At 1 days after operation, drugs and vehicle were administered orally to rats for 14_ days (one time /each day). The spatial learning and memory capability of rats were detected by Morris water maze in the 9-12days after operation. The testing of Morris water was commenced at 40min after drugs treated. The biochemical and pathology assays conducted later (in 24 h) after behavioral testing. The experimental schedule is shown in Fig. 26.

### 5. Morris water maze

The navigation test was performed after icv Aβ (25-35) for 9-12 days by the same method as the before. On the thirteenth day, the platform was removed and the rat was allowed to search for the platform for 30 s (probe test). The time in the platform quadrant and latency time to cross platform location were recorded to measure the spatial learning ability without the influence of chance encounters with the platform.

### 6. Biochemical assays

The methods as the before.

### 7. Statistical analysis

The results were expressed as mean±SEM. The data from training trail in the Morris water maze were analyzed by two way analysis of variance (ANOVA) to detect the difference between groups and over time. The post hoc Dunnett's test was used to test the differences between two groups. Probe trial, biochemical assay, and immunohistochemistry assay were statically analyzed using one-way ANOVA followed by post hoc Dunnett's test. The results were considered to show a significant difference when the p value was less than 0.05.

### Results

### 1. dl-PHPB significantly improved Aβ (25-35)-induced impairments in spatial learning and spatial working memory in rats.

In the navigation test, the rats were trained for 4 days. The latency of each group gradually reduced, indicating the memory capacity for the location of the security platform in each group was growing.

On the 1^{st} day, there was no significant difference in each group. But the escape latency of vehicle group has the longer trend than sham group. In the 2^{nd} day, the escape latency of dl-PHPB group (39mg/kg)- has shorter trend than sham group (P <0.05). From 3^{rd} to 4^{th} days, the escape latency of vehicle group was significantly longer than sham group (P <0.05). However, the escape latency of dl-PHPB group (129 mg/kg) was significantly shorter than vehicle group (P <0.05 or P <0.01). The escape latency of dl-PHPB group (39 mg/kg) has shorter trend than vehicle group. The results shown that dl-PHPB could reduce the escape latency of Aβ (25-35)-induced dementia in dose-dependent manner. Namely, dl-PHPB could improve learning and memory capacity of Aβ (25-35)-induced dementia (Figure 18). For 4 days training, the swimming speed of rats in each group was not significantly different (data not shown), indicating the icv Aβ (25-35) in rats does not affect the physical. Water maze test can reliably reflect the ability of learning and memory in animals.

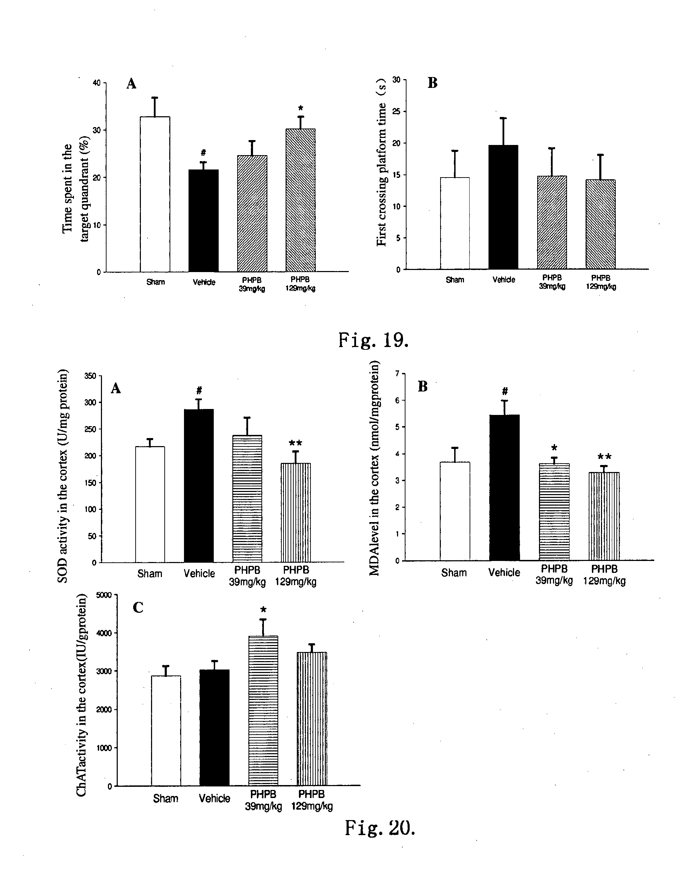

In probe test, animals of the vehicle group have a significantly lower percentage of time in the target quadrant (21.6 ± 1.6%) than the sham group (32.8 ± 4.0%, P <0.05), and dl-PHPB (129. mg/kg) could increase the percentage of time significantly more in the target quadrant (30.2 ± 2.5%) than vehicle group (p<0.05). dl-PHPB (39mg/kg) only had a longer trend (24.6 ± 3.0%) than the vehicle group (Figure 19A). dl-PHPB could increase the time percentage of the target quadrant in dose dependent manner. The animals of vehicle group had the longer trend than the sham group in the time of first cross of the platform location. Compared with the sham group, dl-PHPB also shortens the trend in the time of the first cross platform. If the number of animal in each group is increased, the time of the first cross platform may be appearing significantly different (Figure 19B).

In conclusion, dl-PHPB could improve the short memory and spatial learning impairment of Aβ (25-35)-induced dementia of rats in a dose-dependent manner.

### 2. Effects of dl-PHPB on the SOD, ChAT activity and MDA level of brain tissues from dementia rats.

SOD plays an important role in maintenance of oxygen free radicals balance, can effectively eliminate oxygen free radicals and reduce oxidative damage. MDA is one of the main peroxides. SOD activity may reflect the antioxidant levels in brain tissue, while the level of MDA in brain tissues reflects the situation of lipid peroxidation. The results showed that the SOD activity of Aβ (25-35)-induced dementia rats significantly increased by 32% (286.8±18.3 U /mg protein), compared with sham operation (216.9±14.5 U /mg protein). After oral administration of dl-PHPB 39mg/kg and 129mg/kg for 2 weeks, the SOD activities in the cortex were decreased to 238.2±32.7 and 185.2±21.6U/mg protein, the later had significant difference (P <0.01), compared with vehicle group. dl-PHPB could decrease the cortical SOD activity in dose dependent manner (Figure 20A). In the hippocampus, the SOD activity of Aβ (25-35)-induced dementia rats did not significantly differ; dl-PHPB has not significantly improved SOD activity.

In the cortex, the MDA level of vehicle group (5.43 ± 0.55 nmol / mg protein) was significantly increased, compared with sham operation group (3.69 ± 0.52 nmol / mg protein) (P <0.05). After oral administration dl-PHPB 39mg/kg and 129mg/kg for 2 weeks, the cortex MDA levels significantly decreased to 3.62 ± 0.21 and 3.28 ± 0.25 nmol / mg protein, compared with the sham operation group (P <0.05 and P <0.01 ). The results shown that dl-PHPB could decrease the cortical MDA level of Aβ (25-35)-induced dementia rats in a dose dependent manner (Figure 20B).

Acetylcholine is a neurotransmitter in the central nervous system, mediated by cholinergic nerve signaling. Acetylcholine is closely related with learning and memory, and synthesis by ChAT. So, the activity of ChAT can indirectly reflect levels of acetylcholine, reflecting the status of cholinergic function. In rats with Aβ (25-35)-induced dementia, the cortical ChAT activity did not change significantly, compared with sham operation group. After administration of 2 weeks, 39_ mg/kg of dl-PHPB significantly increased ChAT activity (P <0.05), 129_mg-/-kg is also a strong trend. The results shown that the dl-PHPB may improved the ChAT activity of Aβ (25-35)-induced dementia rats (Figure 20C).

In conclusion, dl-PHPB could decrease the SOD activity and MDA level of Aβ (25-35)-induced dementia rat in a dose dependent manner. dl-PHPB reduces lipid peroxidation, restoration of normal brain tissue oxidation and antioxidant homeostasis. In addition, dl-PHPB maybe increased ChAT activity and improve the cholinergic function in cortex of Aβ (25-35)-induced dementia rats. However, the effect of dl-PHPB for the cortical ChAT activity in normal rats requires further study.

EXAMPLE 3 : The study in SAMP8 mice: dl-PHPB improves the memory and spatial learning capability of SAMP8 mice.

### 1. Materials and methods

dl-PHPB was offered by the department of medical synthetic chemistry of our institute. dl-PHPB was dissolved in PBS. The levels of MDA, SOD , ChAT and ATPase activities were determined with commercial colorimetric assay kits (Nanjing Jiancheng Bioengineering Institute, China). The content of protein in the supernatant was determined by Bradford method using BSA as standard.

### 2.Instrument

The water maze apparatus and DTT-2 jumping apparatus were designed by Institute of Material Medica, Chinese Academy of Medical Sciences. Enzyme mark instrument (MQX 200) was purchased by Bio Tek Instruments. Paraffin section machine (IR2135) purchased by German Leica co., LTD. Thermostatic freezing section machine (620-E) purchased by UK Shandon co., LTD. Automatic microphotography system (Nikon ECLIPSE 80i) purchased by Japanese Nikon Corporation.

### 3. Animals

SAMP8 in 10 months old, male, SPF grade, purchased from Beijing Vital River Laboratory Animal Technology Co., Ltd..

### 4. Treatment Groups and Drug Administration.

SAMP8 were divided into threeexperimental groups randomly : one vehicle control group, and two dl-PHPB-treated groups (50 mg/kg and 160_mg/kg). Drugs and vehicle were administered orally to SAMP8 mice for 35 days (one time /each day). The spatial learning and memory capability of SAMP8 mice were detected by Morris water maze and DTT-2 jumping apparatus in the 31-35days. The testing was commenced at 40_min after drugs treatment. The biochemical and pathology assays conducted later (in 24 h) after behavioral testing. The experimental schedule is shown in Fig. 27.

### 5. Step down test

The step-down avoidance task was performed by using a behavior box (22cm × 15cm × 30cm each). Each testing chamber has 3 Plexiglas black walls, a clear Plexiglas front wall, metal grid floors,and an insulating platform (3cm diameter, 4cm height) which is located at one corner of the testing chamber. The metal grids are connected to the output terminals of an electrical stimulator. For the habituation session, each mouse was gently placed on the insulating platform and was allowed to explore in the testing chamber for 3 min before placing back on the platform again. Monophasic pulses (1 ms, 1 Hz, 36VDC) were continuously delivered for 5min during the training trial. If the mouse steps down from the platform onto the grid floor, the mouse will be subjected to receive an electric shock until returning to the platform. Then, 24 h after the training, the mice were placed on the platform for assessing their long term memory of retention period. The electric shocks were delivered for 5min and the latency to step down on the grid with four paws for the first time (step-down latency) and the numbers of errors subjected to shocks within 5_ min were recorded.

### 6. Water maze test

The water maze consisted of square black opaque plastic box (80cm × 50cm × 20cm), of which four blind-side and a terminal stage. When a black plastic sheet (15cm×20cm) was placed in different locations, there were different starting points and a different number of blind sides. The water maze filled with water at 25± 1°C to a depth 12cm. Mice can be placed in different starting points making experience with a different number of blind sides to the end. The number of errors into the blind side and the time to reach the end platform (escape latency) was recorded.

Each mouse was permitted to stay for 5 s in the water, and allowed 3_min to find the end platform. The escape latency (time to reach the platform) was used to assess acquisition of the water maze task. Sessions were repeated for 4 consecutive days.

### 7. Biochemical assays

The methods as the before.

### 8. Statistical analysis

The results were expressed as mean±SEM. The data from the training trail in the Morris water maze were analyzed by two way analysis of variance (ANOVA) to detect the difference between groups and over time. The post hoc Dunnett's test was used to test the differences between two groups. Probe trial, biochemical assay, and immunohistochemistry assay were statically analyzed using one-way ANOVA followed by post hoc Dunnett's test. The results were considered to show a significant difference when the p value was less than 0.05.

### Results

### 1. dl-PHPB significantly improved impairments in spatial learning and spatial working memory of SAMP8.

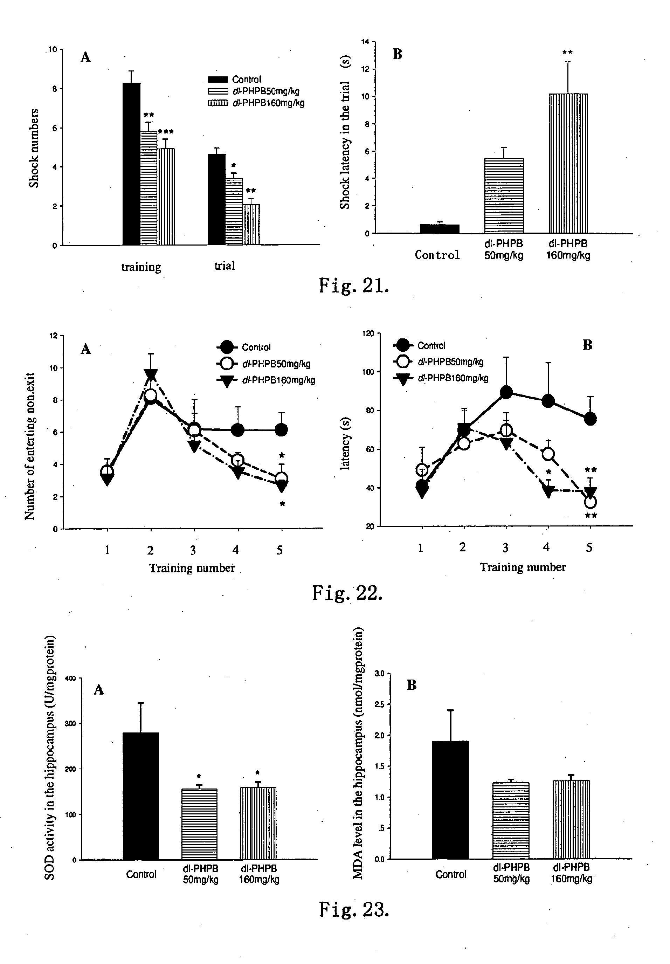

Step down test was a typical experiment to detect the ability of avoidance response in animals. The avoidance response capacity of animals was measured based on the first time (step-down latency) and the numbers of errors subjected to shocks. In the first days of training, dl-PHPB (50 and 160mg/kg) could significantly decrease the numbers of errors subject to shocks (5.8 ± 0.5, 4.9 ± 0.5 Vs 8.3 ± 0.6) in dose dependent manner (P <0.01 and P <0.001). In the second days, the dl-PHPB also significantly decreased the numbers of errors subject to shocks (3.4 ± 0.3,2.1 ± 0.3), compared with vehicle group_(4.6 ± 0.3) (P <0.05 and P <0.01). dl-PHPB could significantly increased step-down latency time (5.5 ± 0.8,10.2 ± 2.4) in a dose dependent manner, compared with the vehicle group_(0.7 ± 0.2) (P <0.01) (Figure 21). The results indicated that dl-PHPB (50 and 160 mg/kg) could enhance the ability of active and passive avoidance response, improving the ability of learning and memory. The water maze test is commonly used to detect the ability of recent memory and spatial learning memory in mice. The ability of learning and memory were evaluated based on the number of errors into the blind side and the time to reach the end platform (escape latency).

In this study, the first and second training and testing sessions include 2 and 3 blind-sides, respectively. However, there are four blind-sides from third to fifth training and testing sessions. During the first three trainings, dl-PHPB administration exerted no significant difference in the number of errors into the blind-side of SAMP8, compared with the vehicle group. In the later three trainings, dl-PHPB could decrease the number of errors into the blind-side of SAMP8, compared with the vehicle group dl-PHPB could significantly decrease the number of errors into the blind-side of SAMP8 in the fourth training, compared with the vehicle group. In the fifth test, the number of errors into the blind-side in SAMP8 treated by dl-PHPB (respectively 3.1 ± 0.9 and 2.7 ± 0.3) was significantly less than the vehicle group (6.1 ± 1.1) (P <0.05).

In the first and second training, escape latency time of each group was not significantly different. In the third training, dl-PHPB has shortened the tendency time in escape latency. However, dl-PHPB in the 160 mg/kg treatment group could significantly shorten the escape latency time in the fourth training (P <0.05). In the last test, dl-PHP in the 50 and 160mg/kg treatment group could significantly shorten the escape latency time (P <0.01), compared with the vehicle group (Figure 22). Thus, dl-PHPB can improve spatial learning and memory defects of SAMP8.

In summary, dl-PHPB at 50 and 160mg/kg could significantly improve short-term memory and spatial learning capability of SAMP8 mice in a dose dependent manner.

### 2. Effects of dl-PHPB on the SOD activity and MDA level of brain tissues from SAMP8.

SOD plays an important role in the maintenance of oxygen free radicals balance, can effectively eliminate oxygen free radicals and reduce oxidative damage. MDA is one of the main peroxides. SOD activity may reflect the antioxidant levels in brain tissue, while the level of MDA in brain tissues reflects the situation of lipid peroxidation. The results showed that the SOD activity of SAMP8 was 279.4±65.7 U /mg protein in the hippocampus. After oral administration of dl-PHPB 50mg/kg and 160mg/kg for 35 days, the SOD activities in the hippocampus were decreased to 15.6.2±7.8 and 158.7±11.4U/mg protein, both having a significant difference -(P <0.05) the compared with vehicle group. dl-PHPB could decrease the SOD activity of hippocampus ina dose dependent manner (Figure 23A). -In the cortex, the SOD activity of dl-PHPB treatment for 35_days did not significantly improved in SOD activity.

After oral administration of dl-PHPB 50mg/kg and 160_mg/kg for 35 days, the MDA levels in the hippocampus were 1.23 ± 0.05 and 1.26 ± 0.09 nmol / mg protein, decreased by 35.3 % and 33.7%, compared with SAMP8, but no statistical significance (Figure 23B). In the cortex, the MDA level of dl-PHPB treated for 35_days did not significantly improved (data not shown).

The results indicated that dl-PHPB could improve disorder of the capacity of anti-oxygenation in the brain, decrease production of lipid peroxidation, and restore the balance of oxidation.

### 2. Effects of dl-PHPB on the ChAT and AChE activity of brain tissues, and ATP level of mitochondria in the cortex from SAMP8.

Acetylcholine is a neurotransmitter in the central nervous system, mediated by cholinergic nerve signaling. Acetylcholine is closely related with learning and memory, and synthesis by ChAT. So, the activity of ChAT can indirectly reflect levels of acetylcholine reflecting the status of cholinergic function. After administration of dl-PHPB for 35 days, dl-PHPB significantly increased ChAT activity (P <0.05) in the hippocampus in dose-dependent manner (Figure 24A). In the cortex, dl-PHPB did not significantly improve ChAT activity in the cortex(data not shown). The results show that dl-PHPB may improve the ChAT activity of SAMP8 (Figure 24B).

Mitochondrial ATPase plays key role in the mitochondrial function. After oral administration dl-PHPB 50mg/kg and 160mg/kg for 35 days, dl-PHPB 160_mg/kg significantly increased the ATPase activity (9.82 ± 0.51 U-/-mg protein), compared with the vehicle group (8.58 ± 0.21 U-/-mg protein). However, the dl-PHPB 50_mg/kg did not show a significant difference (Figure 24C).

In conclusion, dl-PHPB can dose dependently increase the ChAT activity of hippocampus in the SAMP8. The results implied that PHPB might ameliorate the cholinergic function via increasing the ACh content in the hippocampus of SAMP8.

## Claims

1. dl-Potassium 2-(1-hydroxypentyl)-benzoate for use in the prevention, amelioration or treatment of aged dementia or its symptoms, wherein said use is for the prevention, amelioration or treatment of memory loss, cognitive dysfunction, slow thinking or spatial disorientation.

2. The compound for use according to claim 1 for use in the prevention, amelioration or treatment of Alzheimer's, vascular dementia and a combination of both conditions.

3. The compound for use according to claim 1 for use in improving damages induced by oxidative stress in the brain, for use in the enhancement of functions in the cholinergic nerve, in the protection of neurons, and in increasing brain-derived growth factor.

4. The compound for use according to claim 3, wherein the oxidative stress in brain damage reduced by reducing lipid peroxides, by the restoration of normal brain tissue oxidation and by oxidation homeostasis.

5. The compound for use according to claim 4, wherein the antioxidant enzymes are superoxide dismutase and the lipid peroxide is malonaldehyde (MDA).

6. The compound for use according to claim 3, wherein the cholinergic function is improved by increasing choline acetyltransferase (ChAT) activity and inhibiting acetylcholinesterase (AChE) activity.

7. A pharmaceutical composition for use in the prevention, amelioration or treatment of dementia-related signs selected from memory impairment, impaired cognition, impaired thought process and impaired spatial orientation comprising dl-potassium 2-(1-hydroxypentyl)-benzoate in a prevention- or treatment-effective dose and optionally pharmaceutically acceptable carriers and excipients.

8. The pharmaceutical composition for use according to claim 7 for use in the prevention, amelioration or treatment of memory loss, cognitive dysfunction, slow thinking or spatial disorientation.

9. The pharmaceutical composition for use according to claim 7, which is in form of a solution, suspension, emulsion, pill, capsule, powder, controlled-release or sustained release preparation.

10. A composition for use in the prevention, amelioration and treatment of dementia-related signs selected from memory impairment, impaired cognition, impaired thought process and impaired spatial orientation comprising dl-potassium 2-(1-hydroxypentyl)-benzoate in a prevention- or treatment-effective dose.

11. Composition for use according to claim 10 for use in the prevention, amelioration and treatment of Alzheimer's, vascular dementia and both conditions.

12. Composition for use according to claim 11 for use in the prevention, amelioration and treatment of memory loss, cognitive dysfunction, slow thinking or spatial disorientation.

13. Composition for use according to claim 10, in which the dl-potassium 2-(1-hydroxypentyl)-benzoate is formulated for parenteral, oral, focal, intracutaneous, intramusculary, intraperitoneally, subcutaneous or intranasal administration.

14. Composition for use according to claim 10, in which the therapeutically effective dose is from 0.5 to 200 mg/kg body weight, preferably 1-150 mg/kg body weight, more preferably 2-100 mg/kg body weight, in particular 3-50 mg/kg body weight, even more preferably 4-35 mg/kg body weight, and most preferably 5-20 mg/kg body weight.

## Patentansprüche

1. dl-Kalium-2-(1-hydroxypentyl)-benzoat für die Verwendung bei der Verhinderung, Linderung oder Behandlung von Altersdemenz oder deren Symptomen,
wobei die Verwendung der Verhinderung, Linderung oder Behandlung von Gedächtnisverlust, einer kognitiven Funktionsstörung, des langsamen Denkens oder einer räumlichen Orientierungslosigkeit dient.

2. Verbindung für die Verwendung nach Anspruch 1 für die Verwendung bei der Verhinderung, Linderung oder Behandlung der Alzheimer-Krankheit, einer vaskulären Demenz und einer Kombination beider Leiden.

3. Verbindung für die Verwendung nach Anspruch 1 zur Verwendung bei der Verbesserung von Schädigungen, die durch oxidativen Stress im Gehirn hervorgerufen wurden, für die Verwendung bei der Verbesserung von Funktionen in cholinergischen Nerven, beim Schutz von Neuronen und bei der Verstärkung des vom Gehirn stammenden Wachstumsfaktors.

4. Verbindung für die Verwendung nach Anspruch 3,
wobei die Schädigung durch oxidativen Stress im Gehirn durch Verminderung von Lipidperoxiden, durch Wiederherstellung der normalen Oxidation von Hirngewebe und durch Homöostase der Oxidation vermindert wird.

5. Verbindung für die Verwendung nach Anspruch 4,
wobei die Antioxidans-Enzyme Superoxid-Dismutase sind und das Lipidperoxid Malonaldehyd (MDA) ist.

6. Verbindung für die Verwendung nach Anspruch 3,
wobei die cholinergische Funktion durch Erhöhung der Aktivität von Cholinacetyltransferase (ChAT) und Hemmung der Aktivität von Acetylcholinesterase (AChE) verbessert wird.

7. Pharmazeutische Zusammensetzung für die Verwendung bei der Verhinderung, Linderung oder Behandlung von mit Demenz in Zusammenhang stehenden Symptomen, die ausgewählt sind aus einer Gedächtnisstörung, einer beeinträchtigten Wahrnehmung, einem beeinträchtigten Denkprozess und einer beeinträchtigten räumlichen Orientierung,
die dl-Kalium-2-(1-hydoxypentyl)-benzoat in einer für die Verhinderung oder Behandlung wirksamen Dosis und gegebenenfalls pharmazeutisch akzeptable Träger und Korrigentien aufweist.

8. Pharmazeutische Zusammensetzung für die Verwendung nach Anspruch 7, zur Verwendung bei der Verhinderung, Linderung oder Behandlung von Gedächtnisverlust, einer kognitiven Funktionsstörung, des langsamen Denkens oder einer räumlichen Orientierungslosigkeit.

9. Pharmazeutische Zusammensetzung für die Verwendung nach Anspruch 7,
die in Form einer Lösung, Suspension, Emulsion, Pille, Kapsel, eines Pulvers, eines Präparats mit gesteuerter Freisetzung oder mit Langzeitwirkung vorliegt.

10. Zusammensetzung für die Verwendung bei der Verhinderung, Linderung und Behandlung von mit Demenz in Zusammenhang stehenden Symptomen, die aus einer Gedächtnisstörung, einer beeinträchtigten Wahrnehmung, einem beeinträchtigten Denkprozess und einer beeinträchtigten räumlichen Orientierung ausgewählt sind,
die dl-Kalium-2-(1-hydoxypentyl)-benzoat in einer für die Verhinderung oder Behandlung wirksamen Dosis aufweist.

11. Zusammensetzung für die Verwendung nach Anspruch 10,
zur Verwendung bei der Verhinderung, Linderung und Behandlung der Alzheimer-Krankheit, einer vaskulären Demenz und beider Leiden.

12. Zusammensetzung für die Verwendung nach Anspruch 11,
zur Verwendung bei der Verhinderung, Linderung und Behandlung von Gedächtnisverlust, einer kognitiven Funktionsstörung, des langsamen Denkens oder einer räumlichen Orientierungslosigkeit.

13. Zusammensetzung für die Verwendung nach Anspruch 10,
wobei das dl-Kalium-2-(1-hydoxypentyl)-benzoat für die parenterale, orale, fokale, intrakutane, intramuskuläre, intraperitoneale, subkutane oder intranasale Verabreichung formuliert ist.

14. Zusammensetzung für die Verwendung nach Anspruch 10,
wobei die therapeutisch wirksame Dosis 0,5 bis 200 mg/kg Körpergewicht, vorzugsweise 1 bis 150 mg/kg Körpergewicht, stärker bevorzugt 2 bis 100 mg/kg Körpergewicht, insbesondere 3 bis 50 mg/kg Körpergewicht, noch stärker bevorzugt 4 bis 35 mg/kg Körpergewicht und besonders bevorzugt 5 bis 20 mg/kg Körpergewicht beträgt.

## Revendications

1. 2-(1-hydroxypentyl)-benzoate de dl-potassium pour une utilisation dans la prévention, l'amélioration ou le traitement de la démence sénile ou de ses symptômes, ladite utilisation étant pour la prévention, l'amélioration ou le traitement de la perte de mémoire, du dysfonctionnement cognitif, du ralentissement de la pensée ou de la désorientation spatiale.

2. Composé pour une utilisation suivant la revendication 1, pour une utilisation dans la prévention, l'amélioration ou le traitement de la maladie d'Alzheimer, de la démence vasculaire et d'une association de ces deux affections.

3. Composé pour une utilisation suivant la revendication 1, pour une utilisation dans l'amélioration des altérations induites par le stress oxydatif dans le cerveau, pour une utilisation dans l'amélioration de fonctions dans le nerf cholinergique, dans la protection des neurones et dans l'augmentation du facteur de croissance dérivé du cerveau.

4. Composé pour une utilisation suivant la revendication 3, le stress oxydatif dans l'altération cérébrale étant réduit en diminuant les peroxydes lipidiques, par rétablissement de l'oxydation normale du tissu cérébral et par l'homéostase de l'oxydation.

5. Composé pour une utilisation suivant la revendication 4, les enzymes antioxydantes étant la superoxyde-dismutase et le peroxyde lipidique étant le malonaldéhyde (MDA).

6. Composé pour une utilisation suivant la revendication 3, la fonction cholinergique étant améliorée en augmentant l'activité de choline-acétyltransférase (ChAT) et en inhibant l'activité d'acétylcholinestérase (AChE).

7. Composition pharmaceutique pour une utilisation dans la prévention, l'amélioration ou le traitement de signes en rapport avec la démence choisis entre une altération de la mémoire, une altération de la cognition, une altération des processus de pensée et une altération de l'orientation spatiale, comprenant du 2-(1-hydroxypentyl)-benzoate de dl-potassium à une dose efficace à des fins de prévention ou de traitement et éventuellement des supports et excipients pharmaceutiquement acceptables.

8. Composition pharmaceutique pour une utilisation suivant la revendication 7, pour une utilisation dans la prévention, l'amélioration ou le traitement de la perte de mémoire, du dysfonctionnement cognitif, du ralentissement de la pensée ou de la désorientation spatiale.

9. Composition pharmaceutique pour une utilisation suivant la revendication 7, qui est sous forme d'une solution, d'une suspension, d'une émulsion, d'une pilule, d'une capsule, d'une poudre ou d'une préparation à libération contrôlée ou à libération prolongée.

10. Composition pour une utilisation dans la prévention, l'amélioration et le traitement des signes en rapport avec la démence choisis entre l'altération de la mémoire, l'altération de la cognition, l'altération des processus de pensée et l'altération de l'orientation spatiale, comprenant du 2-(1-hydroxypentyl)-benzoate de dl-potassium à une dose efficace à des fins de prévention ou de traitement.

11. Composition pour une utilisation suivant la revendication 10, pour une utilisation dans la prévention, l'amélioration ou le traitement de la maladie d'Alzheimer, de la démence vasculaire et de ces deux affections.

12. Composition pour une utilisation suivant la revendication 11, pour une utilisation dans la prévention, l'amélioration ou le traitement de la perte de mémoire, du dysfonctionnement cognitif, du ralentissement de la pensée ou de la désorientation spatiale.

13. Composition pour une utilisation suivant la revendication 10, dans laquelle le 2-(1-hydroxypentyl)benzoate de dl-potassium est formulé pour l'administration parentérale, orale, focale, intracutanée, intramusculaire, intrapéritonéale, sous-cutanée ou intranasale.

14. Composition pour une utilisation suivant la revendication 10, dans laquelle la dose thérapeutiquement efficace va de 0,5 à 200 mg/kg de poids corporel, avantageusement de 1 à 150 mg/kg de poids corporel, plus avantageusement de 2 à 100 mg/kg de poids corporel, en particulier de 3 à 50 mg/kg de poids corporel, encore plus avantageusement de 4 à 35 mg/kg de poids corporel et de préférence de 5 à 20 mg/kg de poids corporel.
